(19) Europäisches Patentamt
European Patent Office
Office européen des brevets

(11) EP 4 213 842 B1

(12) EUROPEAN PATENT SPECIFICATION

(45) Date of publication and mention of the grant of the patent:
**15.04.2026 Bulletin 2026/16**

(21) Application number: **21869378.6**

(22) Date of filing: **15.09.2021**

(51) International Patent Classification (IPC):
***A61K 31/4245*** (2006.01) ***A61P 35/00*** (2006.01)
***A61P 35/02*** (2006.01) ***A61K 45/06*** (2006.01)

(52) Cooperative Patent Classification (CPC):
(C-Sets available)
**A61K 31/4245; A61K 45/06; A61P 35/00; A61P 35/02** (Cont.)

(86) International application number:
**PCT/JP2021/033855**

(87) International publication number:
**WO 2022/059692 (24.03.2022 Gazette 2022/12)**

# (54) RIBONUCLEOTIDE REDUCTASE INHIBITOR FOR USE IN A METHOD OF TREATING TUMORS

RIBONUKLEOTIDREDUKTASE-INHIBITOR ZUR VERWENDUNG IN DER BEHANDLUNG VON TUMOREN

INHIBITEUR D'UNE RIBONUCLÉOTIDE RÉDUCTASE POUR SON UTILISATION DANS UNE METHODE DE TRAITEMENT DES TUMEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.09.2020 US 202063078844 P**

(43) Date of publication of application:
**26.07.2023 Bulletin 2023/30**

(73) Proprietor: **Taiho Pharmaceutical Co., Ltd.**
**Chiyoda-ku, Tokyo 101-8444 (JP)**

(72) Inventors:
- **UENO Hiroyuki**
  **Tokyo 101-8444 (JP)**
- **HOSHINO Takuya**
  **Tsukuba-shi, Ibaraki 300-2611 (JP)**

(74) Representative: **Wächtershäuser & Hartz Patentanwaltspartnerschaft mbB**
**Weinstraße 8**
**80333 München (DE)**

(56) References cited:
**WO-A1-2019/107386 US-A1- 2013 005 678**
**US-A1- 2020 157 066 US-A1- 2020 157 066**

- **DATABASE EMBASE [online] ELSEVIER SCIENCE PUBLISHERS, AMSTERDAM, NL; 1 December 2019 (2019-12-01), HOSHINO T ET AL: "TAS1553, a novel class of RNR inhibitor, demonstrates synergistic antitumor efficacy in combination with nucleoside analogues", XP002811658, Database accession no. EMB-638177593**
- **HOSHINO T ET AL: "TAS1553, a novel class of RNR inhibitor, demonstrates synergistic antitumor efficacy in combination with nucleoside analogues", MOLECULAR CANCER THERAPEUTICS 20191201 AMERICAN ASSOCIATION FOR CANCER RESEARCH INC. NLD, vol. 18, no. 12 SUPPL 1, 1 December 2019 (2019-12-01), ISSN: 1538-8514**
- **KATO, HIROHISA: "Regimen Management in Kan Drug Therapy", JAPANESE JOURNAL OF DRUG INFORMATICS, vol. 11, no. 4, 1 January 2010 (2010-01-01), pages 9 (217) - 14 (222), XP009535433, ISSN: 1345-1464**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ANONYMOUS: "List of Regimen 28 Leukemia Chemotherapy Regimen", 21 August 2021 (2021-08-21), XP055912538, Retrieved from the Internet <URL:https://www.saiseikai-hp.chuo.fukuoka.jp/cancer/pdf/rejimen/28.pdf>
- MURREN JOHN, MODIANO MANUEL, CLAIRMONT CAROLINE, LAMBERT PAULA, SAVARAJ NIRAMOL, DOYLE TERRY, SZNOL MARIO: "Phase I and Pharmacokinetic Study of Triapine, a Potent Ribonucleotide Reductase Inhibitor, Administered Daily for Five Days in Patients with Advanced Solid Tumors", CLINICAL CANCER RESEARCH, vol. 9, no. 11, 1 January 2003 (2003-01-01), US, pages 4092 - 4100, XP009535050, ISSN: 1078-0432
- GOSS KELLI L., GORDON DAVID J.: "Gene expression signature based screening identifies ribonucleotide reductase as a candidate therapeutic target in Ewing sarcoma", ONCOTARGET, vol. 7, no. 39, 27 September 2016 (2016-09-27), pages 63003 - 63019, XP055912540, DOI: 10.18632/oncotarget.11416


(52) Cooperative Patent Classification (CPC): (Cont.)

C-Sets
**A61K 31/4245, A61K 2300/00;**
**A61K 45/06, A61K 2300/00**

## Description

### Technical Field

The present disclosure relates to a method of treating a patient with RNR-related diseases with a ribonucleotide reductase inhibitor according to a specific dosing regimen. In particular, the present disclosure relates to a method of treating a tumor with a ribonucleotide reductase inhibitor according to a specific dosing regimen.

### Background Art

Ribonucleotide reductase (hereinafter also referred to as "RNR") is composed of a hetero-oligomer of a large subunit M1 and a small subunit M2, and expression of both is required for enzyme activity. RNR recognizes ribonucleoside 5'-diphosphate (hereinafter also referred to as "NDP") as a substrate and catalyzes its reduction to 2'-deoxyribonucleoside 5'-diphosphate (hereinafter also referred to as "dNDP"). RNR is a rate-limiting enzyme in the de novo dNTP synthesis pathway and plays an essential role in DNA synthesis and repair (NPL 1).

The enzymatic activity of RNR is closely related to cell proliferation, and it has been reported that its enzymatic activity is particularly high in cancer (NPL 2). In various types of solid tumors and blood cancers, numerous correlations have been reported with overexpression of M2 subunit of RNR, and its impact on the prognosis of the cancer (NPLs 3 and 4). In addition, cell growth inhibition achieved by inhibition of RNR and an in vivo anti-tumor effect have been reported in cell lines derived from several cancer types and in nonclinical models (NPLs 5 and 6). Thus, it is strongly suggested that RNR is an important target molecule for cancer treatment.

Conventionally, hydroxyurea (hereinafter also referred to as "HU") and 3-aminopyridine-2-carboxaldehyde thiosemicarbazone (hereinafter also referred to as "3-AP") are known to exhibit RNR inhibitory activity. These compounds differ in structure, however, from the sulfonamide compounds of the present disclosure. Although HU has been used clinically for over 30 years, its RNR inhibitory activity is weak and its effect is limited (NPL 7). In addition, resistance to the use of HU is also considered a problem (NPL 8). Meanwhile, 3-AP is capable of chelating to metal ions, especially iron (Fe) ions, thereby inhibiting RNR (NPL 9). However, 3-AP has been suggested as having an off-target effect on various other Fe ion-containing proteins, resulting in side effects such as hypoxia, dyspnea, methemoglobinemia and the like in clinical cases (NPL 10).

Therefore, there is a need to develop an RNR inhibitor which does not chelate with metal ions and can be used in the treatment of diseases associated with RNR, such as cancer.

On the other hand, the compound 5-chloro-2-(N-((1S,2R)-2-(6-fluoro-2,3-dimetylphenyl)-1-(5-oxo-4,5-dihydro-1,3,4-ox adiazol-2-yl)propyl)sulfamoyl)benzamide (referred to herein as Compound A) has been known to have a potent RNR inhibitory activity (PL 1). However, no administration schedules or dosage regimens for Compound A have been described in the art, for example in US 2020/157066 A1.

### Citation List

#### Patent Literature

PTL 1: WO2017/209155

#### Non Patent Literature

NPL 1: Annu. Rev. Biochem. 67, 71-98. (1998)
NPL 2: J. Biol. Chem. 245, 5228-5233. (1970)
NPL 3: Nat. Commun. 5, 3128 doi: 10.1038 / ncomms 4128 (2014)
NPL 4: Clin. Sci. 124, 567-578. (2013)
NPL 5: Expert. Opin. Ther. Targets 17, 1423 - 1437 (2013)
NPL 6: Biochem. Pharmacol. 59, 983-991 (2000)
NPL 7: Biochem. Pharmacol. 78, 1178- 11 85 (2009)
NPL 8: Cancer Res. 54, 3686-3691 (1994)
NPL 9: Pharmacol. Rev. 57, 547-583 (2005)
NPL 10: Future Oncol. 8, 145-150 (2012)

## Summary of Invention

The invention is set out in the appended set of claims. The references to the methods of treatment by therapy of this description are to be interpreted as references to compounds, pharmaceutical compositions and medicaments of the present invention for use in those methods. Aspects of the disclosure include a method of treating a tumor in a patient in need by administering an effective amount of Compound A utilizing a specific administration schedule.

The present inventors have discovered that continuous administration of Compound A shows anti-tumor effects. However, such an administration may at the same time result in one or more unfavorable events or side-effects, such as body weight loss. The inventors conducted extensive studies, and consequently discovered that an intermittent administration of Compound A comprising dosing days with higher doses and non-dosing days without administering Compound A, achieved an anti-tumor effect with fewer unfavorable events and side-effects, compared to consecutive dosing with lower doses. The present disclosure is based on this unexpected and surprising discovery.

In an embodiment, a method of treating a patient with a tumor includes administering an effective amount of 5-chloro-2-(N-((1S,2R)-2-(6-fluoro-2,3-dimetylphenyl)-1-(5-oxo-4,5-dihydro-1,3,4-ox adiazol-2-yl)propyl)sulfamoyl) benzamide or a salt thereof to the patient on an intermittent administration schedule for two weeks comprising dosing 1 to 5 days per week.

## Brief Description of Drawings

[Fig.1]Fig. 1 is a graph illustrating relative tumor volume (RTV) of Control group (○), Continuous Dosing Group (●: 100 mg/kg/day; ◊: 150 mg/kg/day) and QOD Dosing Group (▲: 200 mg/kg/day; ♦: 300 mg/kg/day). Error bars: standard deviations from averages.

[Fig.2]Fig. 2 is a graph showing body weight (BW) change of the Control group (○), the Continuous Dosing group (●: 100 mg/kg/day; ◊: 150 mg/kg/day) and QOD Dosing Group (▲: 200 mg/kg/day; ♦: 300 mg/kg/day). Error bars: standard deviations from averages.

[Fig.3]Fig. 3 is a graph illustrating relative tumor volume (RTV) of Control group (○), Continuous Dosing Group (●: 100 mg/kg/day; ◊: 150 mg/kg/day) and QW Dosing Group (■: 700 mg/kg/day; x:1050 mg/kg/day). Error bars: standard deviations from averages.

[Fig.4]Fig. 4 is a graph showing body weight (BW) change of the Control group (○), the Continuous Dosing group (●: 100 mg/kg/day; ◊: 150 mg/kg/day) and QW Dosing Group (■: 700 mg/kg/day; x:1050 mg/kg/day). Error bars: standard deviations from averages.

[Fig.5]Fig. 5 is a graph illustrating relative tumor volume (RTV) of Control group (○), Continuous Dosing Group (●: 100 mg/kg/day; ◊: 150 mg/kg/day) and 3QW Dosing Group 1 (▲: 233 mg/kg/day; ♦: 350 mg/kg/day). Error bars: standard deviations from averages.

[Fig.6]Fig. 6 is a graph showing body weight (BW) change of the Control group (○), the Continuous Dosing group (●: 100 mg/kg/day; ◊: 150 mg/kg/day) and 3QW Dosing Group 1 (▲: 233 mg/kg/day; ♦: 350 mg/kg/day). Error bars: standard deviations from averages.

[Fig.7]Fig. 7 is a graph illustrating relative tumor volume (RTV) of Control group (○), Continuous Dosing Group (●: 100 mg/kg/day; ◊: 150 mg/kg/day) and 3QW Dosing Group 2 (▲: 233 mg/kg/day; ♦: 350 mg/kg/day). Error bars: standard deviations from averages.

[Fig.8]Fig. 8 is a graph showing body weight (BW) change of the Control group (○), the Continuous Dosing group (●: 100 mg/kg/day; ◊: 150 mg/kg/day) and 3QW Dosing Group 2 (▲: 233 mg/kg/day; ♦: 350 mg/kg/day). Error bars: standard deviations from averages.

[Fig.9]Fig. 9 is a graph illustrating relative tumor volume (RTV) of Control group (○), Continuous Dosing Group (●: 100 mg/kg/day; ◊: 150 mg/kg/day) and 5QW Dosing Group 1 (♦: 140 mg/kg/day; □: 210 mg/kg/day). Error bars: standard deviations from averages.

[Fig.10]Fig. 10 is a graph showing body weight (BW) change of the Control group (○), the Continuous Dosing group (●: 100 mg/kg/day; ◊: 150 mg/kg/day) and 5QW Dosing Group 1 (♦: 140 mg/kg/day; □: 210 mg/kg/day). Error bars: standard deviations from averages.

[Fig.11]Fig. 11 is a graph illustrating relative tumor volume (RTV) of Control group (○), Continuous Dosing Group (●: 100 mg/kg/day; ◊: 150 mg/kg/day) and 5QW Dosing Group 2 (■: 140 mg/kg/day; x: 210 mg/kg/day). Error bars: standard deviations from averages.

[Fig.12]Fig. 12 is a graph showing body weight (BW) change of the Control group (○), the Continuous Dosing group (●: 100 mg/kg/day; ◊: 150 mg/kg/day) and 5QW Dosing Group 2 (■: 140 mg/kg/day; x: 210 mg/kg/day). Error bars: standard deviations from averages.

[Fig.13]Fig. 13 shows Scatterplot indicating the correlation between SLFN11 mRNA expression and cell growth (%) at

100 micro mol/L of Compound A in 11 cell lines.

[Fig.14]Fig. 14 shows the effects of Compound A on the cell growth of A673 cells treated with siRNA against SLFN11 (siSLFN11-1 and siSLFN11-2) or a control siRNA (siControl).

[Fig.15]Fig. 15 shows caspase-3/7 activation by Compound A (10 micro mol/L) in A673 cells treated with siRNA against SLFN11 (siSLFN11-1 and siSLFN11-2).

[Fig.16]Fig. 16 shows SLFN11 expression in A673 cells treated with siRNA. A673 cells were treated with siRNA against SLFN11 (siSLFN11-1 and siSLFN11-2) for 24h, and then harvested and analyzed by immunoblotting.

[Fig.17]Fig. 17 shows the effects of Compound A on the cell growth of NCI-H460 cells treated with siRNA against SLFN11 (siSLFN11-1, siSLFN11-2 and siSLFN11-3) or a control siRNA (siControl).

[Fig.18]Fig. 18 shows the effects of Compound A on the cell growth of CFPAC-1 cells treated with siRNA against SLFN11 (siSLFN11-1, siSLFN11-2 and siSLFN11-3) or a control siRNA (siControl).

[Fig.19]Fig. 19 shows caspase-3/7 activation by Compound A (10 micro mol/L) in NCI-H460 and CFPAC-1 cells treated with siRNA against SLFN11 (siSLFN11-1, siSLFN11-2 and siSLFN11-3).

## Description of Embodiments

Aspects of the disclosure include a method of treating RNR-related diseases by administering an RNR inhibitor on an intermittent administration schedule for two weeks comprising dosing 1 to 5 days per week. In an embodiment, a method of treating RNR-related diseases includes administering 5-chloro-2-(N-((1S,2R)-2-(6-fluoro-2,3-dimetylphenyl)-1-(5-oxo-4,5-dihydro-1,3,4-ox adiazol-2-yl)propyl)sulfamoyl) benzamide ("Compound A") or a salt thereof to a patient in need thereof. In some embodiments, the method involves administering Compound A or a salt thereof on an intermittent administration schedule for two weeks comprising dosing 1 to 5 days per week. The exemplary intermittent administration schedules of the present disclosure reduce unfavorable events or side-effects, such as body weight loss compared to the conventional continuous administration schedule, even though the total dosage of Compound A in two weeks is the same. In some embodiments, the RNR-related diseases are tumors such as acute myeloid leukemia (AML).

5-chloro-2-(N-((1S,2R)-2-(6-fluoro-2,3-dimetylphenyl)-1-(5-oxo-4,5-dihydro-1,3,4-o xadiazol-2-yl)propyl)sulfamoyl)benzamide is depicted in the following structure:

[Chem.1]

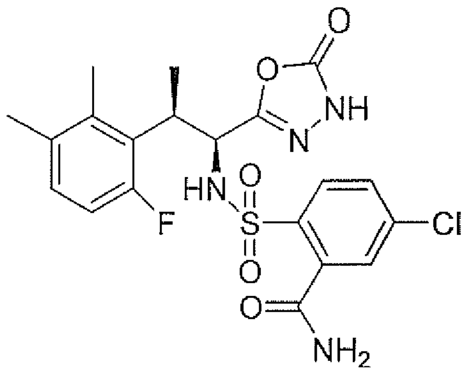

In the present application, the above compound is described as "Compound A." Compound A is described as a compound of Example 5 in International Publication No. WO2017/209155. Compound A can be produced by any known methods in the art, including, but not limited to, those methods described in the International Publication No. WO2017/209155.

The novel methods of treatment described herein exhibit an effect of reducing one or more unfavorable observations, such as side effects, adverse reactions or adverse events, for example, weight loss, while achieving an anti-tumor effect. Also, the novel methods of treatment with an intermittent administration schedule described herein can exhibit a comparable anti-tumor effect, (e.g., same suppression of tumor growth as a continuous administration), and in some embodiments, an intermittent administration schedule (e.g., QOD, 3QW, and 5QW) can exhibit even a higher anti-tumor effect. In example embodiments, Compound A is administered on an intermittent administration schedule for two weeks comprising dosing 1 to 5 days per week. This surprising and unexpected discovery enables longer term administration of Compound A with reduced side effects, etc., which ultimately contributes to a longer survival time and/or a longer period of progression-free survival.

The exemplary administration schedules described herein demonstrate advantages in terms of reduction in one

or more unfavorable events or side-effects.

In the present disclosure, the administration schedule is not particularly limited, as long as it includes an intermittent administration for two weeks comprising dosing 1 to 5 days per week. An administration cycle can be defined as two weeks or more. The cycle can be performed once or repeated twice or more to treat RNR-related diseases.

Exemplary administration schedules may include 4-week (28 day) administration schedules defined as one (1) cycle, the administration can be carried out in one cycle, or more than one cycle, for example, 1, 2, 3, 4, 5, 6, 7, 8, 9, 10 cycles or more. In some embodiments, the administration can be carried out in long-periods comprising several cycles. For example, the administration can be carried out for a period of 6-months with approximately 6 cycles of treatment or administration; a 1-year period with approximately 13 cycles of treatment; a 3-year period with approximately 39 cycles of treatment or more.

In one embodiment, the administration schedule may comprise dosing one day per week. In this case, Compound A may be administered on Day 1 and Day 8 in a 2-week administration schedule (Days 1-14). This schedule may be referred to as "QW" dosing herein.

In another embodiment, the administration schedule may comprise three dosing days and four non-dosing days per week. This schedule may be referred to as "3QW" dosing herein. In this case, the administration schedule may comprise dosing for three consecutive days followed by four non-dosing days in one week. Accordingly, Compound A may be administered on Days 1-3, 8-10 in a 2-week administration schedule (Days 1-14). Alternatively, the administration schedule may comprise dosing every other day three times followed by two non-dosing days in one week. Accordingly, Compound A may be administered on Days 1, 3, 5, 8, 10, and 12 in a 2-week administration schedule (Days 1-14).

In another embodiment, the administration schedule may comprise five dosing days and two non-dosing days per week. This schedule may be referred to as "5QW" dosing herein. In this case, the administration schedule may comprise dosing for five consecutive days followed by two non-dosing days in one week. Accordingly, Compound A may be administered on Days 1-5 and 8-12 in a 2-week administration schedule (Days 1-14). Alternatively, the administration schedule may comprise (a) two consecutive dosing days and one non-dosing day, followed by (b) three consecutive dosing days and one non-dosing day, or may comprise (b) three consecutive dosing days and one non-dosing day, followed by (a) two consecutive dosing days and one non-dosing day, in one week. Accordingly, Compound A may be administered on Days 1-2, 4-6, 8-9 and 11-13 in a 2-week administration schedule (Days 1-14), or may be administered on Days 1-3, 5-6, 8-10 and 12-13 in a 2-week administration schedule.

In another embodiment, the administration schedule may comprise dosing every other day and dosing seven days within two weeks. Accordingly, Compound A may be administered on Days 1, 3, 5, 7, 9, 11, and 13 in a 2-week administration schedule (Days 1-14). This schedule may be referred to as "QOD" dosing herein.

As long as the administration is continued on the defined one cycle, the administration may be stopped after one to several cycles, and the administration may be restarted after a certain period of drug holiday (no administration). Similarly, the administration schedule of the present disclosure may include a schedule having a plurality of periods of drug holidays.

In an embodiment, the administration schedule can include an intermittent administration schedule for two weeks comprising dosing 1 to 5 days per week, and one or more periods of drug holidays. The intermittent administration schedule may be implemented in the dosing period before a drug holiday and in dosing periods after one or more periods of drug holidays.

In another embodiment, the administration schedule can include an intermittent administration schedule for two weeks comprising dosing 1 to 5 days per week, and two periods of drug holidays. The intermittent administration schedule may be implemented in the dosing period before the first period of drug holidays, in the dosing period between the two periods of drug holidays, and in the dosing period after the second period of drug holidays.

In another embodiment, the administration schedule can include an intermittent administration schedule for two weeks comprising dosing 1 to 5 days per week, and two or more periods of drug holidays. The intermittent administration schedule may be implemented in the dosing period before the first period of drug holidays, in the dosing period between the two adjacent periods of drug holidays, and in the dosing period after the last period of drug holidays.

The period of drug holidays is not particularly limited and can be suitably set according to the patient's state, and the like. For example, the period of drug holidays can be within the range of 1 to 35 days. Alternatively, the period of drug holidays can be within the range of 1 to 12 months.

In some embodiments, Compound A or a salt thereof is administered once or more than once each day in any of the dosing days. In a preferred embodiment, Compound A or a salt thereof is administered once per day. In another embodiment, Compound A or a salt thereof is administered twice per day. In another embodiment, Compound A or a salt thereof is administered three times per day.

A typical daily dose of Compound A or salt thereof can be in the range from 100 picograms to 100 milligrams per kilogram of body weight, more typically 10 nanograms to 25 milligrams per kilogram of bodyweight. More typically, a daily dose of Compound A or salt thereof can be in the range from 100 nanograms to 20 milligrams per kilogram of bodyweight although higher or lower doses may be administered where required. For example, the daily dose may be 1 microgram to

20 milligrams of bodyweight, more typically 10 micrograms to 20 milligrams per kilogram of bodyweight, and more typically 100 micrograms to 20 milligrams per kilogram of bodyweight.

Dosages may also be expressed as the amount of drug administered relative to the body surface area of the patient ($mg/m^2$). A typical daily dose of Compound A or salt thereof can be in the range from 3700 $pg/m^2$ to 3700 $mg/m^2$, although higher or lower doses may be administered where required. For example, the daily dose may be 370 $ng/m^2$ to 925 $mg/m^2$, more typically 3700 $ng/m^2$ to 740 $mg/m^2$, although higher or lower doses may be administered where required. For example, the daily dose may be 37 micrograms/$m^2$ to 740 $mg/m^2$, and more typically 370 micrograms/$m^2$ to 740 $mg/m^2$, or 3700 micrograms/$m^2$ to 740 $mg/m^2$.

Compound A or salt thereof of the invention may be administered orally in a range of single doses, for example 0.05 to 5000 mg. Typically, the range may be 10 to 1000 mg. Typical examples of doses include 5, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975 and 1000 mg. The doses may be increased or decreased in a stepwise manner from any dose in the above range (of 0.05 to 5000 mg) in increments/decrements of, for example, 1 mg, 5 mg, 10 mg, 20 mg, 25 mg or 50 mg. The dosage may be altered depending on symptoms of the patients, weight, age, or gender and the like.

In various embodiments, Compound A or a salt there of is administered in the form of a pharmaceutical composition. The pharmaceutical composition comprising Compound A or salts thereof used in the present disclosure can be formulated in accordance with known techniques. See for example, Remington's Pharmaceutical Sciences, Mack Publishing Company, Easton, PA, USA. The pharmaceutical compositions can be in any form suitable for oral, parenteral, topical, intranasal, intrabronchial, sublingual, ophthalmic, otic, rectal, intra-vaginal, or transdermal administration. Of these, oral administration is preferred. Where the compositions are intended for parenteral administration, they can be formulated for intravenous, intramuscular, intraperitoneal, subcutaneous administration or for direct delivery into a target organ or tissue by injection, infusion or other means of delivery. The delivery can be by bolus injection, short-term infusion or longer-term infusion and can be via passive delivery or through the utilization of a suitable infusion pump or syringe driver.

Compound A or a salt thereof used in the present disclosure may be in the form of crystals, including co-crystals. Single crystals and polymorphic crystal mixtures are included within the scope of Compound A or a salt thereof. Such crystals can be produced by crystallization according to a crystallization method known in the art. Compound A or a salt thereof may be a solvate (e.g., a hydrate) or a non-solvate. Any of such forms are included within the scope of the compound of the present disclosure or a salt thereof. A crystalline form including co-crystal of Compound A is disclosed in the International Publication No. WO2019/106579.

Compound A may be labeled with an isotope (e.g., $^{3}H$, $^{14}C$, $^{35}S$, and $^{125}I$), and such a labeled compound and a salt thereof is also included within the scope of Compound A or a salt thereof used in the present disclosure.

The salts of Compound A used in the present disclosure refer to common salts used in the field of organic chemistry. Examples of such salts include base addition salts, and acid addition salts. The salts of Compound A are preferably pharmaceutically acceptable salts.

In an embodiment, Compound A or salts thereof are in the free form of Compound A (i.e., not a salt of Compound A). In an embodiment, Compound A is in the form of a co-crystal of Compound A and benzoic acid.

Due to their excellent RNR inhibitory activity, Compound A or salts thereof used in the present disclosure are useful as a pharmaceutical preparation for preventing and treating RNR-related diseases. Accordingly, the administration schedule of the present disclosure is useful for treating RNR-related diseases. "RNR" herein includes human or non-human RNR, preferably human RNR.

Examples of "RNR-related diseases" or diseases "characterized by expression and/or activity of RNR", which terms are used interchangeably herein, include diseases, the incidence of which can be reduced, and symptoms of which can be remitted, relieved, and/or completely cured by eliminating, suppressing, and/or inhibiting RNR function. Examples of such diseases include a tumor including a malignant tumor. The type of malignant tumor to be treated by the Compound A or a salt thereof is not particularly limited. Examples of such malignant tumors include glandular tumors, carcinoid tumors, undifferentiated carcinomas, angiosarcoma, adenocarcinoma, gastrointestinal cancers (e.g., colorectal cancers ("CRC"), including colon cancer and rectal cancer, biliary cancers including gall bladder cancer and bile duct cancer, anal cancer, esophageal cancer, gastric (stomach) cancer, gastrointestinal carcinoid tumor(s), gastrointestinal stromal tumor(s) ("GIST"), liver cancer, duodenal cancer and small intestine cancer), lung cancers (e.g., non-small cell lung cancer ("NSCLC"), squamous-cell lung carcinoma, large-cell lung carcinoma, small cell lung carcinoma, mesothelioma and other lung cancers, such as bronchial tumors and pleuropulmonary blastoma), urological cancers (e.g., kidney (renal) cancer, transitional cell cancer ("TCC") of kidney, TCC of the renal pelvis and ureter ("PDQ"), bladder cancer, urethral cancer and prostate cancer), head and neck cancers (e.g., eye cancer, retinoblastoma, intraocular melanoma, hypopharyngeal cancer, pharyngeal cancer, laryngeal cancer, laryngeal papillomatosis, metastatic squamous neck cancer with occult primary, oral (mouth) cancer, lip cancer, throat cancer, oropharyngeal cancer, esthesioneuroblastoma, nasal cavity and paranasal sinus cancer, nasopharyngeal cancer, and salivary gland cancer), endocrine cancers (e.g., thyroid

cancer, parathyroid cancer, multiple endocrine neoplasia syndromes, thymoma and thymic carcinoma, pancreatic cancers including pancreatic ductal adenocarcinoma ("PDAC"), pancreatic neuroendocrine tumors and islet cell tumors), breast cancers (extrahepatic ductal carcinoma in situ ("DCIS"), lobular carcinoma in situ ("LCIS"), triple negative breast cancer, and inflammatory breast cancer), male and female reproductive cancers (e.g., cervical cancer, ovarian cancer, endometrial cancer, uterine sarcoma, uterine cancer, vaginal cancer, vulvar cancer, gestational trophoblastic tumor ("GTD"), extragonadal germ cell tumor, extracranial germ cell tumor, germ cell tumor, testicular cancer and penile cancer), brain and nervous system cancers (e.g., astrocytomas, brain stem glioma, brain tumor, craniopharyngioma, central nervous system ("CNS") cancer, chordomas, ependymoma, embryonal tumors, neuroblastoma, paraganglioma and atypical teratoid), skin cancers (e.g., basal cell carcinoma ("BCC"), squamous cell skin carcinoma ("SCC"), Merkel cell carcinoma and melanoma), tissue and bone cancers (e.g., soft-tissue sarcoma, rhabdomyosarcoma, fibrous histiocytoma of bone, Ewing sarcoma, malignant fibrous histiocytoma of bone ("MFH"), osteosarcoma and chondrosarcoma), cardiovascular cancers (e.g., heart cancer and cardiac tumors), appendix cancers, childhood and adolescent cancers (e.g., adrenocortical carcinoma childhood, midline tract carcinoma, hepatocellular carcinoma ("HCC"), hepatoblastoma and Wilms' tumor) and viral-induced cancers (e.g., HHV-8 related cancers (Kaposi sarcoma) and HIV/AIDS related cancers). In some embodiments, the cancer is lung cancer, pancreatic cancer, or colorectal cancer. Other preferable examples include solid tumors.

Examples of such malignant tumors also include, but are not limited to, hematological and plasma cell malignancies (e.g., cancers that affect blood, bone marrow and/or lymph nodes) such as multiple myeloma, leukemias and lymphomas, myelodysplastic syndromes and myeloproliferative disorders (myeloproliferative neoplasms), and myelodysplastic/myeloproliferative neoplasms (MDS/MPN) overlap syndromes. Leukemias include, without limitation, acute lymphoblastic leukemia ("ALL"), acute myelogenous (myeloid) leukemia ("AML"), chronic lymphocytic leukemia ("CLL"), chronic myelogenous leukemia ("CML"), acute monocytic leukemia ("AMoL"), hairy cell leukemia, and/or other leukemias. Lymphomas include, without limitation, Hodgkin's lymphoma and non-Hodgkin's lymphoma ("NHL"). In some embodiments, NHL is B-cell lymphomas and/or T-cell lymphomas. In some embodiments, NHL includes, without limitation, diffuse large B-cell lymphoma ("DLBCL"), small lymphocytic lymphoma ("SLL"), chronic lymphocytic leukemia ("CLL"), mantle cell lymphoma ("MCL"), Burkitt's lymphoma, cutaneous T-cell lymphoma including mycosis fungoides and Sezary syndrome, AIDS-related lymphoma, follicular lymphoma, lymphoplasmacytic lymphoma (Waldenstrom's macroglobulinemia ("WM")), primary central nervous system (CNS) lymphoma and/ or other lymphomas.

Preferable examples include myeloid neoplasms, such as acute myeloid leukemia (AML). In one aspect, the RNR-related diseases to be targeted by the present disclosure is acute myeloid leukemia (AML), including relapsed or refractory (R/R) acute myeloid leukemia (AML). In another aspect, the RNR-related diseases to be targeted by the present disclosure is myelodysplastic syndromes. In another aspect, the RNR-related diseases to be targeted by the present disclosure is myeloproliferative disorders (myeloproliferative neoplasms). In another aspect, the RNR-related diseases to be targeted by the present disclosure is myelodysplastic/myeloproliferative neoplasms (MDS/MPN) overlap syndromes. Another preferable examples include solid tumors.

In one aspect, the RNR-related diseases to be targeted by the present disclosure is a SLFN11 (Schlafen Family Member 11)-positive tumor. It has been reported that SLFN11 contributes to the sensitivity of Ewing sarcoma cells to inhibition of RNR (Oncotarget, (2016 Sep 27) Vol. 7, No. 39, pp. 63003-63019) and that SLFN11 binds replication forks in response to replication stress (Mol. Cell (2018) Vol 69, Issue 3, pp 371-384.e6). However, the correlation between SLFN11 expression in tumor cells and the effects of Compound A or a salt thereof against tumors has not been clarified. The present disclosure indicates for the first time that SLFN11-positive tumor can be targeted by Compound A or a salt thereof, as demonstrated by Examples below.

In the present disclosure, "SLFN11" means human or non-human SLFN11, and preferably human SLFN11. Further, "SLFN11" includes an isoform. An example of the nucleotide sequence of human SLFN11 gene include the sequence represented by NCBI Reference Sequence: NM_001104587.2. An example of the amino acid sequence of human SLFN11 protein include the sequence represented by NCBI Reference Sequence: NP_001098057.1. The nucleotide sequence and the amino acid sequence shown above may comprise polymorphic mutation. The polymorphic mutation is, for example, silent mutation that does not cause a change in amino acid residue; or a mutation by deletion, substitution, or insertion of 1 or more (for example, about 1 to 5, or 1 to 3) amino acid residues compared to the amino acid sequence of wild-type SLFN11.

In the present disclosure, "SLFN11-positive" means that the condition under which SLFN11 is expressed in a higher level than a normal level. SLFN11 positivity can be determined by measuring the expression level. There are no particular restrictions on what is measured in measuring the expression level, as long as the expression level can be quantitatively or semiquantitatively measured. Examples include mRNA expression level and protein expression level.

The expression level can be measured from a sample of the subject. "sample" includes not only a biological sample (e.g., cells, tissues, organs, body fluids (blood, lymph fluid, and the like), digestive fluid, urine), but also a nucleic acid extract (e.g., genomic DNA extracts, mRNA extracts, cDNA preparation or cRNA preparation prepared from mRNA extracts, and the like) or a protein extract obtained from these biological samples. Further, the sample may be subjected to

a formalin fixation treatment, an alcohol fixation treatment, a freezing treatment, or a paraffin embedding treatment. The sample preferably contains tumor cells. The method for obtaining a biological sample can be suitably selected, depending on the type of biological sample.

The mRNA expression level, when selected for the measurement, can be measured using a primer or probe that specifically hybridizes with SLFN11 mRNA in accordance with a conventionally used technique for measuring the expression level of mRNA, such as Northern blotting, RT-PCR, real-time PCR, DNA microarray, in situ hybridization, and RNA-seq. The detection device can be any known device (GeneChip, microarray, etc.). The primer and the probe can be prepared based on the known DNA or mRNA sequence information of SLFN11 (e.g., human SLFN11 mRNA NCBI Reference Sequence: NM_001104587.2) by a generally known method as a polynucleotide that specifically hybridizes with DNA or mRNA of human SLFN11.

The expression level of protein, when selected for the measurement, can be measured using an antibody that specifically recognizes the protein of SLFN11 in accordance with a conventionally used measurement method, such as ELISA, Western blotting, immunohistochemical staining and immunofluorescence-based method. The antibody used for the measurement is not particularly limited, insofar as it specifically recognizes the protein of SLFN11 (anti-SLFN11 antibody). Examples include immunoglobulins (IgA, IgD, IgE, IgG, IgM, IgY, etc.), Fab fragments, F(ab')2 fragments, single-chain antibody fragments (scFv), single-domain antibodies, diabodies, and the like. Examples of these antibodies include, but are not limited to, polyclonal antibodies, and monoclonal antibodies (mouse antibodies, llama antibodies, chicken antibodies, rabbit antibodies, donkey antibodies, chimeric antibodies, humanized antibodies, human antibodies, and the like). These antibodies may be prepared by using various known methods. The preparation methods are not particularly limited. Examples of known methods include a method of inoculating a full-length or a fragment of SLFN11 protein into an animal to activate the immune system of the animal, collecting a serum of the animal, and obtaining anti-SLFN11 polyclonal antibodies; or a method of obtaining anti-SLFN11 monoclonal antibodies by a hybridoma method, a phage display method, or the like. Alternatively, commercially available antibodies can also be used. Examples include anti-SLFN11 antibody (#sc515071) (Santa Cruz Biotechnology, Inc.).

The expression level of the protein in the staining in immunohistochemical or immunofluorescence-based method can be calculated from the staining ratio (positive cell occupancy) and the staining intensity. The protein expression level in the staining may be, for example, the numerical value of the H-score method obtained by the following calculation formula (Am. J. Clin. Pathol., 90 (3): 233-9 (1988)).

[Math.1]

$$\text{H-score} = \Sigma\,(\text{Staining Intensity} \times \text{Positive Cell Occupancy (\%)})$$

(0: No Staining, 1: Weak Staining Intensity, 2: Medium -Level Staining Intensity,

3: Strong Staining Intensity)

In addition to the H-score method, values obtained by other scoring methods, such as the Allred method (Allred DC et al., Mod. Pathol., 11: 155-68 (1998)), in which values are obtained according to the following calculation formula: Allred score = positive cell occupancy score + staining intensity score (positive cell occupancy score; 0: No Staining, 1: less than 1%, 2: not less than 1% and less than 10%, 3: not less than 10% and less than 1/3, 4: not less than 1/3 and less than 2/3, 5: not less than 2/3) (Staining Intensity; 0: No Staining, 1: Weak Staining Intensity, 2: Medium-Level Staining Intensity, 3: Strong Staining Intensity); the J-Score method (Kenbikyo, 44 (1): 30-34 (2009)) (J-Score 0: No Staining, J-Score 1: positive cell occupancy of less than 1%, J-Score 2: positive cell occupancy of not less than 1% and less than 10%, J-Score 3: positive cell occupancy of not less than 10%), which performs scoring using only positive cell occupancy (%); etc., can be used.

The expression level of protein, when selected for the measurement, can be measured by a method with using an antibody, for example, a method using mass spectrometry (MS), e.g. LC-MS/MS or MALDI-TOF MS, ESI Q MS, ESI-IT MS or combinations thereof, that can be optionally used in combination with two dimensional electrophoresis (2-DE).

The phrase "the condition under which SLFN11 is expressed in a higher level than a normal level" means that the expression level of SLFN11 in a sample of a tumor patient is relatively high; in one embodiment, the expression level of SLFN11 is equal to or higher than a predetermined cut-off point.

The cut-off point as used herein varies depending on various conditions, such as the type of the object to be measured and the type of measurement method, and the cut-off point is not limited to a particular value. A specific cut-off point can be determined in accordance with various statistical analysis techniques using premeasured expression levels of SLFN11 of tumor patients. Examples include the mean value and the median value of the SLFN11 expression level in

tumor patients; a cut-off point to separate an SLFN11 high-expression group and an SLFN11 low-expression group among tumor patients, which is a value at which the P value becomes minimum and less than the standard value (e.g., a value at which the P value is not more than 0.1 or not more than 0.05) in a logrank test with regard to therapeutic effects (tumor shrinkage effects, progression-free survival extension effects, and the like) of a chemotherapy using an antitumor agent comprising Compound A or a salt thereof in an SLFN11 high-expression group and an SLFN11 low-expression group; a cut-off point to separate an SLFN11 high-expression group and an SLFN11 low-expression group among tumor patients, which is a value determined from the relationship between the SLFN11 expression level in a patient who was subjected to a chemotherapy using an antitumor agent comprising Compound A or a salt thereof and the presence or absence of therapeutic effects (tumor shrinkage effects, progression-free survival extension effects, and the like) of the chemotherapy using Compound A or a salt thereof to a certain extent or more so that the sum of the sensitivity and the specificity becomes maximum based on ROC (Receiver Operating Characteristic) analysis; and a cut-off point to separate an SLFN11 high-expression group and an SLFN11 low-expression group among tumor patients, which is a value at which the P value becomes minimum and less than the standard value (e.g., a value at which the P value is not more than 0.1 or not more than 0.05) in a chi-square test with regard to therapeutic effects (tumor shrinkage effects, progression-free survival extension effects, and the like) of a chemotherapy using an antitumor agent comprising an RNR inhibitor in an SLFN11 high-expression group and an SLFN11 low-expression group, and the like. Among these, the mean value and the median value of the SLFN11 expression level in tumor patients are preferable, and the mean value of the SLFN11 expression level in tumor patients is more preferable.

One example of the cut-off point is that H-score equal to or larger than 1 indicates SLFN11 positive. The other examples of the cut off value is that H-score equal to or larger than 30 indicates SLFN11 positive, that H-score equal to or larger than 100 indicates SLFN11 positive, that H-score equal to or larger than 200 indicates SLFN11 positive, and so on. Such a cut-off point can be determined based on measurement result obtained from a sample from a patient administered with Compound A or a salt thereof and efficacy of Compound A or a salt thereof in the patient.

SLFN11 positivity can also be determined by methods that can show results scientifically similar to those of the methods mentioned above.

Examples of SLFN11-positive tumor includes not only tumor that tests positive at the primary stage, but also tumor that tests positive for SLFN11 at least once at any stage in the progression of tumor, including metastasis and recurrence.

When Compound A or a salt thereof is used in a pharmaceutical preparation, a pharmaceutical carrier can be added, if required, thereby forming a suitable dosage form according to prevention and treatment purposes. Examples of acceptable dosage forms include oral preparations, injections, suppositories, ointments, patches, and the like. Of these, oral preparations are preferable. Such dosage forms can be formed by methods conventionally known to persons skilled in the art.

With respect to the pharmaceutical carrier, various conventional organic or inorganic carrier materials can be used as preparation materials. For example, such materials can be blended as an excipient, binder, disintegrant, lubricant, or coating agent in solid preparations; or as a solvent, solubilizing agent, suspending agent, isotonizing agent, pH adjuster, buffer, or soothing agent in liquid preparations. Moreover, pharmaceutical preparation additives, such as antiseptics, antioxidants, colorants, taste-masking or flavoring agents, and stabilizers, can also be used, if required.

Exemplary oral solid preparations can be prepared as follows. After an excipient is added optionally with a binder, disintegrant, lubricant, colorant, taste-masking or flavoring agent, etc., to Compound A, the resulting mixture is formulated into tablets, coated tablets, granules, powders, capsules, or the like by methods known in the art.

Examples of excipients include lactose, sucrose, D-mannitol, glucose, starch, calcium carbonate, kaolin, microcrystalline cellulose, and silicic acid anhydride. Examples of binders include water, ethanol, 1-propanol, 2-propanol, simple syrup, liquid glucose, liquid $\alpha$-starch, liquid gelatin, D-mannitol, carboxymethyl cellulose, hydroxypropyl cellulose, hydroxypropyl starch, methyl cellulose, ethyl cellulose, shellac, calcium phosphate, polyvinylpyrrolidone, and the like. Examples of disintegrators include dry starch, sodium alginate, powdered agar, sodium hydrogen carbonate, calcium carbonate, sodium lauryl sulfate, stearic acid monoglyceride, lactose, and the like. Examples of lubricants include purified talc, sodium stearate, magnesium stearate, borax, polyethylene glycol, and the like. Examples of colorants include titanium oxide, iron oxide, and the like. Examples of taste-masking or flavoring agents include sucrose, bitter orange peel, citric acid, tartaric acid, and the like.

When a liquid preparation for oral administration is prepared, a taste-masking agent, a buffer, a stabilizer, a flavoring agent, and the like may be added to Compound A; and the resulting mixture may be formulated into an oral liquid preparation, syrup, elixir, etc., according to methods known in the art.

Examples of taste-masking or flavoring agents may be the same as those mentioned above. Examples of buffers include sodium citrate and the like. Examples of the stabilizer include tragacanth, gum arabic, gelatin, and the like. As necessary, these preparations for oral administration may be coated according to methods known in the art with an enteric coating or other coating for the purpose of, for example, persistence of effects. Examples of such coating agents include hydroxypropyl methylcellulose, ethyl cellulose, hydroxymethyl cellulose, hydroxypropyl cellulose, polyoxyethylene glycol,

and TWEEN$^{(R)}$ 80.

When an injection is prepared, a pH adjuster, a buffer, a stabilizer, an isotonizing agent, a topical anesthetic, and the like may be added to Compound A; and the resulting mixture may be formulated into subcutaneous, intramuscular, and intravenous injections according to methods known in the art.

Examples of pH adjusters and buffers include sodium citrate, sodium acetate, sodium phosphate, and the like. Examples of stabilizers include sodium pyrosulfite, EDTA, thioglycolic acid, thiolactic acid, and the like. Examples of topical anesthetics include procaine hydrochloride, lidocaine hydrochloride, and the like. Examples of isotonizing agents include sodium chloride, glucose, D-mannitol, glycerin, and the like.

The amount of Compound A or a salt thereof to be formulated in each dosage unit forms can be, in general, per dosage unit form, from about 0.05, 0.1, 1, 5, 10 , 20 or 25 to about 100, 500 or 1000 mg for an oral dosage, from about 0.01 or 0.1 to about 200, 300 or 500 mg for injection, and from about 1, 5 or 10 to about 100, 500 or 1000 mg for suppositories with the proviso that these amounts may be altered depending on the symptoms of the patients or based on the dosage form used.

In an embodiment, Compound A or salts thereof can be administered in a single dose or in a single cycle of an exemplary administration schedule. In exemplary embodiments, Compound A or salts thereof can be administered in combination with other drug(s) in accordance with an exemplary administration schedule. When Compound A or salts thereof are administered in combination with other drugs, Compound A or salts thereof can be administered on the same day and/or at the same timing as such other drug(s), or, Compound A can be administered on a different day and/or at a different timing from such other drug(s). Such other drug(s) can be administered continuously, sporadically or intermittently during the administration schedule of Compound A or salts thereof.

Using Compound A or a salt thereof in combination with one or more other anti-tumor agents may enhance the anti-tumor effect. Accordingly, the present disclosure also encompasses an administration schedule using Compound A or a salt thereof in such a combinatory manner. Compound A or a salt thereof and one or more other anti-tumor agents may be administered in a single preparation (i.e., a combination drug) or in two or more separate preparations to be administered in combination.

The anti-tumor effect can be evaluated as, for example, reduced tumor volume, tumor growth stasis, or prolonged survival time.

In an embodiment, the administration schedule includes administering an anti-tumor formulation comprising the combination of Compound A or a salt thereof and one or more other anti-tumor agents. In another embodiment, the administration schedule includes administering an anti-tumor effect potentiator for an anti-tumor agent, the potentiator comprising Compound A or a salt thereof as an active ingredient.

The other anti-tumor agents are not particularly limited. Examples of additional anti-cancer agents include chemotherapeutic agents (e.g., cytotoxic agents), immunotherapeutic agents, hormonal and anti-hormonal agents, targeted therapy agents, and anti-angiogenesis agents. Many anti-cancer agents can be classified within one or more of these groups. While certain anti-cancer agents have been categorized within a specific group(s) or subgroup(s) herein, many of these agents can also be listed within one or more other group(s) or subgroup(s), as would be presently understood in the art. It is to be understood that the classification herein of a particular agent into a particular group is not intended to be limiting. Many anti-cancer agents are presently known in the art and can be used in combination with the compounds of the present disclosure.

Further, an agent can be an agonist, antagonist, allosteric modulator, toxin or, more generally, may act to inhibit or stimulate its target (e.g., receptor or enzyme activation or inhibition). For example, suitable for use are one or more agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor "c-met".

In various embodiments, the additional anti-cancer agent is a chemotherapeutic agent, an immunotherapeutic agent, a hormonal agent, an anti-hormonal agent, a targeted therapy agent, or an anti-angiogenesis agent (or angiogenesis inhibitor). In an embodiment, the additional anti-cancer agent is selected from the group consisting of a chemotherapeutic agent, a mitotic inhibitor, a plant alkaloid, an alkylating agent, an anti-metabolite, a platinum analog, an enzyme, a topoisomerase inhibitor, a retinoid, an aziridine, an antibiotic, a hormonal agent, an anti-hormonal agent, an anti-estrogen, an anti-androgen, an anti-adrenal, an androgen, a targeted therapy agent, an immunotherapeutic agent, a biological response modifier, a cytokine inhibitor, a tumor vaccine, a monoclonal antibody, an immune checkpoint inhibitor, an anti-PD-1 agent, an anti-PD-L1 agent, a colony-stimulating factor, an immunomodulator, an immunomodulatory imide (IMiD), an anti-CTLA4 agent, an anti-LAGl agent, an anti-OX40 agent, a GITR agonist, a CAR-T cell, a BiTE, a signal transduction inhibitor, a growth factor inhibitor, a tyrosine kinase inhibitor, an EGFR inhibitor, a histone deacetylase (HDAC) inhibitor, a histone methyltransferase inhibitor, a proteasome inhibitor, a cell-cycle inhibitor, an anti-angiogenesis agent, a matrix-metalloproteinase (MMP) inhibitor, a hepatocyte growth factor inhibitor, a TOR inhibitor, a KDR inhibitor, a VEGF inhibitor, a HIF-1$\alpha$ inhibitor a HIF-2$\alpha$ inhibitor, a fibroblast growth factor (FGF) inhibitor, a RAF inhibitor, a MEK inhibitor, an ERK inhibitor, a PI3K inhibitor, an AKT inhibitor, an MCL-1 inhibitor, a BCL-2 inhibitor, an SHP2 inhibitor, a HER-2 inhibitor, a

BRAF-inhibitor, a gene expression modulator, an autophagy inhibitor, an apoptosis inducer, an antiproliferative agent, and a glycolysis inhibitor.

In various embodiments, the additional anti-cancer agent(s) is a chemotherapeutic agent. Non-limiting examples of chemotherapeutic agents include mitotic inhibitors and plant alkaloids, alkylating agents, anti-metabolites, platinum analogs, enzymes, topoisomerase inhibitors, retinoids, aziridines, and antibiotics.

Non-limiting examples of mitotic inhibitors and plant alkaloids include taxanes such as cabazitaxel, docetaxel, larotaxel, ortataxel, paclitaxel, and tesetaxel; demecolcine; epothilone; eribulin; etoposide (VP- 16); etoposide phosphate; navelbine; noscapine; teniposide; thaliblastine; vinblastine; vincristine; vindesine; vinflunine; and vinorelbine.

Non-limiting examples of alkylating agents include nitrogen mustards such as chlorambucil, chlornaphazine, cholophosphamide, cytophosphane, estramustine, ifosfamide, mannomustine, mechlorethamine, mechlorethamine oxide hydrochloride, melphalan, novembichin, phenesterine, prednimustine, tris(2-chloroethyl)amine, trofosfamide, and uracil mustard; alkyl sulfonates such as busulfan, improsulfan, and piposulfan; nitrosoureas such as carmustine, chlorozotocin, fotemustine, lomustine, nimustine, ranimustine, streptozotocin, and TA-07; ethylenimines and methylamelamines such as altretamine, thiotepa, triethylenemelamine, triethylenethiophosphaoramide, trietylenephosphoramide, and trimethylolomelamine; ambamustine; bendamustine; dacarbazine; etoglucid; irofulven; mafosfamide; mitobronitol; mitolactol; pipobroman; procarbazine; temozolomide; treosulfan; and triaziquone.

Non-limiting examples of anti-metabolites include folic acid analogues such as aminopterin, denopterin, edatrexate, methotrexate, pteropterin, raltitrexed, and trimetrexate; purine analogs such as 6-mercaptopurine, 6-thioguanine, fludarabine, forodesine, thiamiprine, and thioguanine; pyrimidine analogs such as 5-fluorouracil (5-FU), 6-azauridine, ancitabine, azacytidine, capecitabine, carmofur, cytarabine, decitabine, dideoxyuridine, doxifiuridine, doxifluridine, enocitabine, floxuridine, galocitabine, gemcitabine, and sapacitabine; 3-aminopyridine-2-carboxaldehyde thiosemicarbazone; broxuridine; cladribine; cyclophosphamide; cytarabine; emitefur; hydroxyurea; mercaptopurine; nelarabine; pemetrexed; pentostatin; tegafur; and troxacitabine.

Non-limiting examples of platinum analogs include carboplatin, cisplatin, dicycloplatin, heptaplatin, lobaplatin, nedaplatin, oxaliplatin, satraplatin, and triplatin tetranitrate.

Non-limiting examples of enzymes include asparaginase and pegaspargase.

Non-limiting examples of topoisomerase inhibitors include acridine carboxamide, amonafide, amsacrine, belotecan, elliptinium acetate, exatecan, indolocarbazole, irinotecan, lurtotecan, mitoxantrone, razoxane, rubitecan, SN-38, sobuzoxane, and topotecan.

Non-limiting examples of retinoids include alitretinoin, bexarotene, fenretinide, isotretinoin, liarozole, RII retinamide, and tretinoin.

Non-limiting examples of aziridines include benzodopa, carboquone, meturedopa, and uredopa.

Non-limiting examples of antibiotics include intercalating antibiotics; anthracenediones; anthracycline antibiotics such as aclarubicin, amrubicin, daunomycin, daunorubicin, doxorubicin, epirubicin, idarubicin, menogaril, nogalamycin, pirarubicin, and valrubicin; 6-diazo-5-oxo- L-norleucine; aclacinomysins; actinomycin; authramycin; azaserine; bleomycins; cactinomycin; calicheamicin; carabicin; carminomycin; carzinophilin; chromomycins; dactinomycin; detorubicin; esorubicin; esperamicins; geldanamycin; marcellomycin; mitomycins; mitomycin C; mycophenolic acid; olivomycins; novantrone; peplomycin; porfiromycin; potfiromycin; puromycin; quelamycin; rebeccamycin; rodorubicin; streptonigrin; streptozocin; tane-spimycin; tubercidin; ubenimex; zinostatin; zinostatin stimalamer; and zorubicin.

In various embodiments, the additional anti-cancer agent(s) is a hormonal and/or anti-hormonal agent (i.e., hormone therapy). Non-limiting examples of hormonal and anti-hormonal agents include anti-androgens such as abiraterone, apalutamide, bica-lutamide, darolutamide, enzalutamide, flutamide, goserelin, leuprolide, and nilutamide; anti-estrogens such as 4- hydroxy tamoxifen, aromatase inhibiting 4(5)-imidazoles, EM-800, fosfestrol, fulvestrant, keoxifene, LY 117018, onapristone, raloxifene, tamoxifen, toremifene, and trioxifene; anti-adrenals such as aminoglutethimide, dexaminoglutethimide, mitotane, and trilostane; androgens such as calusterone, dromostanolone propionate, epitiostanol, mepitiostane, and testolactone; abarelix; anastrozole; cetrorelix; deslorelin; exemestane; fadrozole; finasteride; formestane; histrelin (RL 0903); human chorionic gonadotropin; lanreotide; LDI 200 (Milkhaus); letrozole; leuprorelin; mifepristone; nafarelin; nafoxidine; osaterone; prednisone; thyrotropin alfa; and triptorelin.

In various embodiments, the additional anti-cancer agent(s) is an immunotherapeutic agent (i.e., immunotherapy). Non-limiting examples of immunotherapeutic agents include biological response modifiers, cytokine inhibitors, tumor vaccines, monoclonal antibodies, immune checkpoint inhibitors, colony-stimulating factors, and immunomodulators.

Non-limiting examples of biological response modifiers, including cytokine inhibitors (cytokines) such as interferons and interleukins, include interferon alfa/ interferon alpha such as interferon alfa-2, interferon alfa-2a, interferon alfa-2b, interferon alfa-nl, interferon alfa-n3, interferon alfacon-1, peginterferon alfa-2a, peginterferon alfa-2b, and leukocyte alpha interferon; interferon beta such as interferon beta-1a, and interferon beta-1b; interferon gamma such as natural interferon gamma-1a, and interferon gamma-1b; aldesleukin; interleukin-1 beta; interleukin-2; oprelvekin; sonermin; tasonermin; and virulizin.

Non-limiting examples of tumor vaccines include APC 8015, AVICINE, bladder cancer vaccine, cancer vaccine (Biomira), gastrin 17 immunogen, Maruyama vaccine, melanoma lysate vaccine, melanoma oncolysate vaccine (New York Medical College), melanoma vaccine (New York University), melanoma vaccine (Sloan Kettering Institute), TICE(R) BCG (Bacillus Calmette-Guerin), and viral melanoma cell lysates vaccine (Royal Newcastle Hospital).

Non-limiting examples of monoclonal antibodies include abagovomab, ade-catumumab, aflibercept, alemtuzumab, blinatumomab, brentuximab vedotin, CA 125 MAb (Biomira), cancer MAb (Japan Pharmaceutical Development), daclizumab, daratumumab, denosumab, edrecolomab, gemtuzumab zogamicin, HER- 2 and Fc MAb (Medarex), ibritumomab tiuxetan, idiotypic 105AD7 MAb (CRC Technology), idiotypic CEA MAb (Trilex), ipilimumab, lintuzumab, LYM-1 -iodine 131 MAb (Techni clone), mitumomab, moxetumomab, ofatumumab, polymorphic epithelial mucin-yttrium 90 MAb (Antisoma), ranibizumab, rituximab, and trastuzumab.

Non-limiting examples of immune checkpoint inhibitors include anti-PD-1 agents or antibodies such as cemiplimab, nivolumab, and pembrolizumab; anti-PD-L1 agents or antibodies such as atezolizumab, avelumab, and durvalumab; anti-CTLA-4 agents or antibodies such as ipilumumab; anti-LAG1 agents; and anti-OX40 agents.

Non-limiting examples of colony-stimulating factors include darbepoetin alfa, epoetin alfa, epoetin beta, filgrastim, granulocyte macrophage colony stimulating factor, lenograstim, leridistim, mirimostim, molgramostim, nartograstim, peg-filgrastim, and sargramostim.

Non-limiting examples of additional immunotherapeutic agents include BiTEs, CAR-T cells, GITR agonists, imiquimod, immunomodulatory imides (IMiDs), mismatched double stranded RNA (Ampligen), resiquimod, SRL 172, and thymalfasin.

In various embodiments, the additional anti-cancer agent(s) is a targeted therapy agent (i.e., targeted therapy). Targeted therapy agents include, for example, monoclonal antibodies and small molecule drugs. Non-limiting examples of targeted therapy agents include signal transduction inhibitors, growth factor inhibitors, tyrosine kinase inhibitors, EGFR inhibitors, histone deacetylase (HDAC) inhibitors, histone methyltransferase inhibitor, proteasome inhibitors, cell-cycle inhibitors, angiogenesis inhibitors, matrix-metalloproteinase (MMP) inhibitors, hepatocyte growth factor inhibitors, TOR inhibitors, KDR inhibitors, VEGF inhibitors, fibroblast growth factors (FGF) inhibitors, MEK inhibitors, ERK inhibitors, PI3K inhibitors, AKT inhibitors, MCL-1 inhibitors, BCL-2 inhibitors, SHP2 inhibitors, HER-2 inhibitors, BRAF-inhibitors, gene expression modulators, autophagy inhibitors, apoptosis inducers, antiproliferative agents, and glycolysis inhibitors.

Non-limiting examples of signal transduction inhibitors include tyrosine kinase inhibitors, multiple-kinase inhibitors, anlotinib, avapritinib, axitinib, dasatinib, dovitinib, imatinib, lenvatinib, lonidamine, nilotinib, nintedanib, pazopanib, pegvisomant, ponatinib, vandetanib, and EGFR inhibitory agents.

Non-limiting examples of EGFR inhibitory agents include small molecule antagonists of EGFR such as afatinib, brigatinib, erlotinib, gefitinib, lapatinib, and osimertinib; and antibody-based EGFR inhibitors, including any anti-EGFR antibody or antibody fragment that can partially or completely block EGFR activation by its natural ligand. Antibody-based EGFR inhibitory agents may include, for example, those described in Modjtahedi, H., et al., 1993, Br. J. Cancer 67:247-253; Teramoto, T., et al., 1996, Cancer 77:639-645; Goldstein et al, 1995, Clin. Cancer Res. 1 : 1311-1318; Huang, S. M., et al., 1999, Cancer Res. 15:59(8): 1935-40; and Yang, X., et al., 1999, Cancer Res. 59: 1236-1243; monoclonal antibody Mab E7.6.3 (Yang, 1999 supra); Mab C225 (ATCC Accession No. HB-8508), or an antibody or antibody fragment having the binding specificity thereof; specific antisense nucleotide or siRNA; afatinib, cetuximab; matuzumab; necitumumab; nimotuzumab; panitumumab; and zalutumumab.

Non-limiting examples of histone deacetylase (HDAC) inhibitors include belinostat, panobinostat, romidepsin, and vorinostat.

Non-limiting examples of histone methyltransferase inhibitors include ezh2 inhibitors.

Non-limiting examples of proteasome inhibitors include bortezomib, carfilzomib, ixazomib, marizomib (salinosporamide a), and oprozomib.

Non-limiting examples of cell-cycle inhibitors, such as CDK inhibitors, include abemaciclib, alvocidib, palbociclib, and ribociclib.

In various embodiments, the additional anti-cancer agent(s) is an anti-angiogenic agent (or angiogenesis inhibitor) including, but not limited to, matrix-metalloproteinase (MMP) inhibitors; VEGF inhibitors; EGFR inhibitors; TOR inhibitors such as everolimus and temsirolimus; PDGFR kinase inhibitory agents such as crenolanib; HIF-1$\alpha$ inhibitors such as PX 478; HIF-2$\alpha$ inhibitors such as belzutifan and the HIF-2$\alpha$ inhibitors described in WO 2015/035223; fibroblast growth factor (FGF) or FGFR inhibitory agents such as B-FGF and RG 13577; hepatocyte growth factor inhibitors; KDR inhibitors; anti-Ang1 and anti-Ang2 agents; anti-Tie2 kinase inhibitory agents; Tek antagonists (US 2003/0162712; US 6,413,932); anti-TWEAK agents (US 6,727,225); ADAM distintegrin domain to antagonize the binding of integrin to its ligands (US 2002/0042368); anti-eph receptor and/or anti-ephrin antibodies or antigen binding regions (US 5,981,245; 5,728,813; 5,969,110; 6,596,852; 6,232,447; and 6,057,124); and anti-PDGF-BB antagonists as well as antibodies or antigen binding regions specifically binding to PDGF-BB ligands.

Non-limiting examples of matrix-metalloproteinase (MMP) inhibitors include MMP-2 (matrix-metalloproteinase 2) inhibitors, MMP-9 (matrix-metalloproteinase 9) inhibitors, prinomastat, RO 32-3555, and RS 13-0830. Examples of

useful matrix metalloproteinase inhibitors are described, for example, in WO 96/33172, WO 96/27583, EP 1004578 , WO 98/07697, WO 98/03516, WO 98/34918, WO 98/34915, WO 98/33768, WO 98/30566, EP 0606046, EP 0931788, WO 90/05719, WO 99/52910, WO 99/52889, WO 99/29667, WO 1999/007675 , EP 1786785, EP 1181017, US 2009/0012085 , US 5,863,949, US 5,861,510, and EP 0780386. Preferred MMP-2 and MMP-9 inhibitors are those that have little or no activity inhibiting MMP-1. More preferred, are those that selectively inhibit MMP-2 and/or MMP-9 relative to the other matrix-metalloproteinases (i.e., MAP-1, MMP-3, MMP-4, MMP-5, MMP-6, MMP- 7, MMP- 8, MMP-10, MMP-11, MMP-12, and MMP-13).

Non-limiting examples of VEGF and VEGFR inhibitory agents include be-vacizumab, cediranib, CEP 7055, CP 547632, KRN 633, orantinib, pazopanib, pegaptanib, pegaptanib octasodium, semaxanib, sorafenib, sunitinib, VEGF antagonist (Borean, Denmark), and VEGF-TRAP™.

The additional anti-cancer agent(s) may also be another anti-angiogenic agent including, but not limited to, 2-methoxyestradiol, AE 941, alemtuzumab, alpha-D148 Mab (Amgen, US), alphastatin, anecortave acetate, angiocidin, angiogenesis inhibitors, (SUGEN, US), angiostatin, anti-Vn Mab (Crucell, Netherlands), atiprimod, axitinib, AZD 9935, BAY RES 2690 (Bayer, Germany), BC 1 (Genoa Institute of Cancer Research, Italy), beloranib, benefin (Lane Labs, US), cabozantinib, CDP 791 (Celltech Group, UK), chondroitinase AC, cilengitide, combretastatin A4 prodrug, CP 564959 (OSI, US), CV247, CYC 381 (Harvard University, US), E 7820, EHT 0101, en-dostatin, enzastaurin hydrochloride, ER-68203-00 (IVAX, US), fibrinogen-E fragment, Flk-1 (ImClone Systems, US), forms of FLT 1 (VEGFR 1), FR-111142, GCS-100, GW 2286 (GlaxoSmithKline, UK), IL-8, ilomastat, IM-862, irsogladine, KM-2550 (Kyowa Hakko, Japan), lenalidomide, lenvatinib, MAb alpha5beta3 integrin, second generation (Applied Molecular Evolution, USA and MedImmune, US), MAb VEGF (Xenova, UK), marimastat, maspin (Sosei, Japan), metastatin, motuporamine C, M-PGA, ombrabulin, OXI4503, PI 88, platelet factor 4, PPI 2458, ramucirumab, rBPI 21 and BPI-derived antiangiogenic (XOMA, US), regorafenib, SC-236, SD-7784 (Pfizer, US), SDX 103 (University of California at San Diego, US), SG 292 (Telios, US), SU-0879 (Pfizer, US), TAN-1120, TBC-1635, tesevatinib, tetrathiomolybdate, thalidomide, thrombospondin 1 inhibitor, Tie-2 ligands (Regeneron, US), tissue factor pathway inhibitors (EntreMed, US), tumor necrosis factor-alpha inhibitors, tumstatin, TZ 93, urokinase plasminogen activator inhibitors, vadimezan, vandetanib, vasostatin, vatalanib, VE-cadherin-2 antagonists, xanthorrhizol, XL 784 (Exelixis, US), ziv-aflibercept, and ZD 6126.

In various embodiments, the additional anti-cancer agent(s) is an additional active agent that disrupts or inhibits RAS-RAF-ERK or PI3K-AKT-TOR signaling pathways or is a PD-1 and/or PD-L1 antagonist. In various embodiments, the additional anti-cancer agent(s) is a RAF inhibitor, EGFR inhibitor, MEK inhibitor, ERK inhibitor, PI3K inhibitor, AKT inhibitor, TOR inhibitor, MCL-1 inhibitor, BCL-2 inhibitor, SHP2 inhibitor, proteasome inhibitor, or immune therapy, including monoclonal antibodies, immunomodulatory imides (IMiDs), anti-PD-1, anti-PDL-1, anti-CTLA4, anti-LAGl, and anti-OX40 agents, GITR agonists, CAR-T cells, and BiTEs.

Non-limiting examples of RAF inhibitors include dabrafenib, encorafenib, regorafenib, sorafenib, and vemurafenib.

Non-limiting examples of MEK inhibitors include binimetinib, CI-1040, co-bimetinib, PD318088, PD325901, PD334581, PD98059, refametinib, selumetinib, and trametinib.

Non-limiting examples of ERK inhibitors include LY3214996, LTT462, MK-8353, SCH772984, ravoxertinib, ulixertinib, and an ERKi as described in WO 2017/068412.

Non-limiting examples of PI3K inhibitors include 17-hydroxywortmannin analogs (e.g., WO 06/044453); AEZS-136; alpelisib; AS-252424; buparlisib; CAL263; co-panlisib; CUDC-907; dactolisib (WO 06/122806); demethoxyviridin; duvelisib; GNE-477; GSK1059615; IC87114; idelalisib; INK1117; LY294002; Palomid 529; paxalisib; perifosine; PI-103; PI-103 hydrochloride; pictilisib (e.g., WO 09/036,082; WO 09/055,730); PIK 90; PWT33597; SF1126; sonolisib; TGI 00-115; TGX-221; XL147; XL-765; wortmannin; and ZSTK474.

Non-limiting examples of AKT inhibitors include Akt-1-1 (inhibits Aktl) (Barnett et al. (2005) Biochem. J., 385 (Pt. 2), 399-408); Akt-1-1,2 (Barnett et al. (2005) Biochem. J. 385 (Pt. 2), 399-408); API-59CJ-Ome (e.g., Jin et al. (2004) Br. J. Cancer 91, 1808-12); 1-H-imidazo[4,5-c]pyridinyl compounds (e.g., WO05011700); indole-3-carbinol and derivatives thereof (e.g., U.S. Patent No. 6,656,963; Sarkar and Li (2004) J Nutr. 134(12 Suppl), 3493S-3498S); perifosine, Dasmahapatra et al. (2004) Clin. Cancer Res. 10(15), 5242-52, 2004); phosphatidylinositol ether lipid analogues (e.g., Gills and Dennis (2004) Expert. Opin. Investig. Drugs 13, 787-97); triciribine (Yang et al. (2004) Cancer Res. 64, 4394-9); imidazooxazone compounds including trans-3-amino-1-methyl-3-(4-(3-phenyl-5H-imidazo[1,2-c]pyrido[3,4-e][1,3]oxazin-2-yl)phenyl)-cyclobutanol hydrochloride (WO 2012/137870); afuresertib; capivasertib; MK2206; and patasertib.

Non-limiting examples of TOR inhibitors include deforolimus; ATP-competitive TORC1/TORC2 inhibitors, including PI-103, PP242, PP30, and Torin 1; TOR inhibitors in FKBP12 enhancer, rapamycins and derivatives thereof, including temsirolimus, everolimus, WO 9409010; rapalogs (e.g. as disclosed in WO 98/02441 and WO 01/14387, e.g. AP23573, AP23464, or AP23841); 40-(2-hydroxyethyl)rapamycin, 40-[3- hydroxy(hydroxymethyl)methylpropanoate]-rapamycin ; 40-epi-(tetrazolyl)-rapamycin (also called ABT578); 32-deoxorapamycin; 16-pentynyloxy-32(S)-dihydrorapanycin, and other derivatives disclosed in WO 05/005434; derivatives disclosed in US 5,258,389, WO 94/090101, WO

92/05179, US 5,118,677, US 5,118,678, US 5,100,883, US 5,151,413, US 5,120,842, WO 93/111130, WO 94/02136, WO 94/02485, WO 95/14023, WO 94/02136, WO 95/16691, WO 96/41807, WO 96/41807 and US 5,256,790; and phosphorus-containing rapamycin derivatives (e.g., WO 05/016252).

Non-limiting examples of MCL-1 inhibitors include AMG-176, MIK665, and S63845.

Additional non-limiting examples of anti-cancer agents that are suitable for use include 2-ethylhydrazide, 2,2',2"-trichlorotriethylamine, ABVD, aceglatone, acemannan, aldophosphamide glycoside, alpharadin, amifostine, aminolevulinic acid, anagrelide, ANCER, ancestim, anti-CD22 immunotoxins, antitumorigenic herbs, apaziquone, arglabin, arsenic trioxide, azathioprine, BAM 002 (Novelos), bcl-2 (Genta), bestrabucil, biricodar, bisantrene, bromocriptine, brostallicin, bryostatin, buthionine sulfoximine, calyculin, cell-cycle nonspecific antineoplastic agents, cel-moleukin, clodronate, clotrimazole, cytarabine ocfosfate, DA 3030 (Dong-A), de-fofamine, denileukin diftitox, dexrazoxane, diaziquone, dichloroacetic acid, dilazep, discodermolide, docosanol, doxercalciferol, edelfosine, eflornithine, EL532 (Elan), elfomithine, elsamitrucin, eniluracil, etanidazole, exisulind, ferruginol, folic acid replenisher such as frolinic acid, gacytosine, gallium nitrate, gimeracil/oteracil/tegafur combination (S-1), glycopine, histamine dihydrochloride, HIT diclofenac, HLA-B7 gene therapy (Vical), human fetal alpha fetoprotein, ibandronate, ibandronic acid, ICE chemotherapy regimen, imexon, iobenguane, IT-101 (CRLX101), laniquidar, LC 9018 (Yakult), leflunomide, lentinan, levamisole + fluorouracil, lovastatin, lucanthone, masoprocol, melarsoprol, metoclopramide, miltefosine, miproxifene, mitoguazone, mitozolomide, mopidamol, motexafin gadolinium, MX6 (Galderma), naloxone + pentazocine, nitracrine, nolatrexed, NSC 631570 octreotide (Ukrain), olaparib, P-30 protein, PAC-1, palifermin, pamidronate, pamidronic acid, pentosan polysulfate sodium, phenamet, picibanil, pixantrone, platinum, podophyllinic acid, porfimer sodium, PSK (Polysaccharide-K), rabbit antithymocyte polyclonal antibody, ras-buriembodiment, retinoic acid, rhenium Re 186 etidronate, romurtide, samarium (153 Sm) lexidronam, sizofiran, sodium phenylacetate, sparfosic acid, spirogermanium, strontium-89 chloride, suramin, swainsonine, talaporfin, tariquidar, tazarotene, tegafururacil, temoporfin, tenuazonic acid, tetrachlorodecaoxide, thrombopoietin, tin ethyl etiopurpurin, tirapazamine, TLC ELL-12, tositumomab-iodine 131, trifluridine and tipiracil combination, troponin I (Harvard University, US), urethan, valspodar, verteporfin, zoledronic acid, and zosuquidar.

In an embodiment, the administration schedule includes administering an anti-tumor formulation comprising the combination of Compound A or a salt thereof and radiation therapy to treat cancer. Techniques for administering radiation therapy are known in the art.

Radiation therapy can be administered through one of several methods, or a combination of methods, including, without limitation, external-beam therapy, internal radiation therapy, implant radiation, stereotactic radiosurgery, systemic radiation therapy, radiotherapy and permanent or temporary interstitial brachy therapy. The term "brachytherapy," as used herein, refers to radiation therapy delivered by a spatially confined radioactive material inserted into the body at or near a tumor or other proliferative tissue disease site. The term is intended, without limitation, to include exposure to radioactive isotopes (e.g., At-211, I-131, I -125, Y-90, Re-186, Re-188, Sm- 153, Bi-212, P-32, and radioactive isotopes of Lu). Suitable radiation sources for use as a cell conditioner of the present disclosure include both solids and liquids. By way of non-limiting example, the radiation source can be a radionuclide, such as I-125, I -131, Yb-169, Ir-192 as a solid source, I-125 as a solid source, or other radionuclides that emit photons, beta particles, gamma radiation, or other therapeutic rays. The radioactive material can also be a fluid made from any solution of radionuclide(s) (e.g., a solution of I-125 or I-131), or a radioactive fluid can be produced using a slurry of a suitable fluid containing small particles of solid radionuclides, such as Au-198, Y-90. Moreover, the radionuclide(s) can be embodied in a gel or radioactive microspheres.

The preparations of the one or more other anti-tumor agents also include drug delivery system (DDS) preparations for them. For example, "paclitaxel" includes albumin-bound paclitaxel (e.g., Abraxane), paclitaxel micelles (e.g., NK105), and the like; and "cisplatin" includes cisplatin micelles (e.g., NC-6004) and the like.

The administration schedule of the present disclosure is applicable for preventing diseases or disorders characterized by the expression of RNR. It is also applicable for administration in pre-surgical operation or post-surgical operation, or as an adjuvant therapy or post-adjuvant therapy.

## Examples

The present disclosure is described below in more detail with reference to Examples. However, the scope of the present disclosure is not limited to these Examples.

### Example 1

Anti-tumor Effect and body weight change in QOD Dosing Regimen

Human acute myelocytic leukemia (AML) cell line MV-4-11 cells (American Type Culture Collection CRL-9591™) were implanted in the right thorax of male nude mice (CLEA Japan, Inc.). After implantation of the tumor, the major axis (mm) and minor axis (mm) of the tumor were measured, and tumor volume (TV) was calculated for each mouse. Then, the

mice were assigned to the following groups, Control group (○), Continuous Dosing Group (●: 100 mg/kg/day; ◊: 150 mg/kg/day) and QOD Dosing Group (▲: 200 mg/kg/day; ♦: 300 mg/kg/day), so that the average TV of each group was equivalent. The day in which the grouping (n=5) was conducted was taken as Day 0.

5-chloro-2-(N-((1S,2R)-2-(6-fluoro-2,3-dimetylphenyl)-1-(5-oxo-4,5-dihydro-1,3,4-o xadiazol-2-yl)propyl)sulfamoyl)benzamide (Compound A) was suspended in 5 mg/mL hypromellose solution to provide appropriate test samples for 100 mg/kg, 150 mg/kg, 200 mg/kg, and 300 mg/kg dosing regimen. Animals of the Control group were not treated.

To mice in the Continuous Dosing Group, 100 mg/kg/day or 150mg/kg/day of Compound A was orally administered once daily from Day 1 to Day 14. The total dosage for mice in the Continuous Dosing Group administered 100 mg/kg/day (Group C-1) was 1400 mg/kg, and the total dosage for mice in the Continuous Dosing Group administered 150 mg/kg/day (Group C-2) was 2100 mg/kg.

To mice in the QOD Dosing Group, 200 mg/kg/day or 300 mg/kg/day of Compound A was orally administered once every other day for two weeks (Days 1, 3, 5, 7, 9, 11 and 13). The total dosage for mice in the QOD Dosing Group administered 200 mg/ kg/day (Group QOD-1) was 1400 mg/kg, and the total dosage for mice in the QOD Dosing Group administered 300 mg/kg/day (Group QOD-2) was 2100 mg/kg.

On Days 4, 8, 11 and 15, using the following formula: TV ($mm^3$) = (major axis x minor $axis^2$) / 2, relative tumor volume (RTV) was calculated as an index of anti-tumor effect during the dosing period for each group (Fig. 1).

As shown in Fig. 1, both the Continuous Dosing Group and QOD Dosing Group showed the effect of reducing tumor volume compared to the Control Group on Day 15. When comparing Group C-1 and Group QOD-1 with the total dosage of 1400 mg/ kg, the effect of reducing tumor volume in Group QOD-1 was higher than Group C-1, and the difference is statistically significant (t-test $p<0.05$). When comparing Group C-2 and Group QOD-2 with the total dosage of 2100 mg/kg, the effect of reducing tumor volume in Group QOD-2 and Group C-2 was similar, and the difference is not statistically significant.

On the other hand, the body weight (BW) was measured on Days 4, 8, 11 and 15, and the average body weight change (BW change, %) relative to the body weight on Day 0 was calculated by the following formula, as an index of toxicity during the dosing period: BW change (%) = [(BW on Day n) - (BW on Day 0)] / (BW on Day 0) x 100. The results are shown in Fig. 2.

As shown in Fig. 2, body weight loss was observed in the Continuous Dosing Group and QOD Dosing Group. When comparing Group C-1 and Group QOD-1 with the total dosage of 1400 mg/kg, a statistically significant difference could not be observed. However, when comparing Group C-2 and Group QOD-2 with the total dosage of 2100 mg/kg, the recovery indicated by the BW change % values in Group QOD-2 was better with a statistically significant difference compared with Group C-2 on Day 15 (t-test $p<0.05$). This result indicates improved body weight reduction as an index of side-effects of Compound A administration in the intermittent dosing regimen (QOD Dosing Group) over the continuous daily dosing regimen (Continuous Dosing Group).

The above results revealed that the intermittent regimen (dosing every other day) is a less toxic dosing schedule and a very useful method that exhibits higher or similar anti-tumor effects while avoiding toxicity resulting from Compound A administration.

**Example 2**

Anti-tumor Effect and body weight change in QW Dosing Regimen

Similar to Example 1, anti-tumor effect and body weight change were studied in mice harboring MV-4-11 cells among the following five groups: Control group (○), Continuous Dosing Group (●: 100 mg/kg/day; ◊: 150 mg/kg/day) and QW Dosing Group (■: 700 mg/kg/day; x:1050 mg/kg/day).

5-chloro-2-(N-((1S,2R)-2-(6-fluoro-2,3-dimetylphenyl)-1-(5-oxo-4,5-dihydro-1,3,4-o xadiazol-2-yl)propyl)sulfamoyl)benzamide (Compound A) was suspended in 5 mg/mL hypromellose solution to provide appropriate test samples for 100 mg/kg, 150 mg/kg, 700 mg/kg, and 1050 mg/kg dosing regimen. Animals of the Control group were not treated.

To mice in the Continuous Dosing Group, 100 mg/kg/day or 150mg/kg/day of Compound A was orally administered once daily from Day 1 to Day 14. The total dosage for mice in the Continuous Dosing Group administered 100 mg/kg/day (Group C-1) was 1400 mg/kg, and the total dosage for mice in the Continuous Dosing Group administered 150 mg/kg/day (Group C-2) was 2100 mg/kg.

To mice in the QW Dosing Group, 700 mg/kg/day or 1050 mg/kg/day of Compound A was orally administered once per week for two weeks (Days 1 and 8). The total dosage for mice in the QW Dosing Group administered 700 mg/kg/day (Group QW-1) was 1400 mg/kg, and the total dosage for mice in the QW Dosing Group administered 1050 mg/kg/day (Group QW-2) was 2100 mg/kg.

On Days 4, 8, 11 and 15, relative tumor volume (RTV) was calculated as an index of anti-tumor effect during the dosing period for each group (Fig. 3).

As shown in Fig. 3, both the Continuous Dosing Group and QW Dosing Group showed the effect of reducing

tumor volume compared to the Control Group on Day 15. When comparing Group C-1 and Group QW-1 with the total dosage of 1400 mg/ kg, the effect of reducing tumor volume in Group QW-1 was similar to Group C-1, and the difference is not statistically significant. When comparing Group C-2 and Group QW-2 with the total dosage of 2100 mg/kg, the effect of reducing tumor volume in Group QW-2 and Group C-2 was similar, and the difference is not statistically significant.

On the other hand, as shown in Fig. 4, body weight loss was observed in the Continuous Dosing Group and QOD Dosing Group. When comparing Group C-1 and Group QW-1 with the total dosage of 1400 mg/kg, the recovery indicated by the BW change % values in Group QW-1 on Day 15 was better with a statistically significant difference than Group C-1 (t-test, $p<0.05$). Similarly, when comparing Group C-2 and Group QW-2 with the total dosage of 2100 mg/kg, the recovery indicated by the BW change % values in Group QW-2 on Day 15 was better with Group QW-2 having a statistically significant difference relative to Group C-2 (t-test, $p<0.05$). This result indicates improved body weight reduction as an index of side-effects of Compound A administration in the intermittent dosing regimen (QW Dosing Group) over the continuous daily dosing regimen (Continuous Dosing Group).

The above results revealed that the intermittent regimen (dosing once per week) is a less toxic dosing schedule with comparable anti-tumor effect relative to a conventional continuous dosing schedule, and a very useful method that exhibits anti-tumor effects while avoiding toxicity resulting from Compound A administration.

**Example 3**

Anti-tumor Effect and body weight change in 3QW Dosing Regimen 1

Similar to Example 1, anti-tumor effect and body weight change were studied in mice harboring MV-4-11 cells among the following five groups: Control group (○), Continuous Dosing Group (●: 100 mg/kg/day; ◊: 150 mg/kg/day) and 3QW Dosing Group 1 (▲: 233 mg/kg/day; ♦: 350 mg/kg/day).

5-chloro-2-(N-((1S,2R)-2-(6-fluoro-2,3-dimetylphenyl)-1-(5-oxo-4,5-dihydro-1,3,4-o xadiazol-2-yl)propyl)sulfamoyl)benzamide (Compound A) was suspended in 5 mg/mL hypromellose solution to provide appropriate test samples for 100 mg/kg, 150 mg/kg, 233 mg/kg, and 350 mg/kg dosing regimen. Animals of the Control group were not treated.

To mice in Continuous Dosing Group, 100 mg/kg/day or 150mg/kg/day of Compound A was orally administered once daily from Day 1 to Day 14. The total dosage for mice in the Continuous Dosing Group administered 100 mg/kg/day (Group C-1) was 1400 mg/kg, and the total dosage for mice in the Continuous Dosing Group administered 150 mg/kg/day (Group C-2) was 2100 mg/kg.

To mice in the 3QW Dosing Group 1, 233 mg/kg/day or 350 mg/kg/day of Compound A was orally administered three days consecutively per week for two weeks (Days 1-3 and 8-10). The total dosage for mice in the 3QW Dosing Group 1 administered 233 mg/kg/day (Group 3QW1-1) was 1400 mg/kg, and the total dosage for mice in the 3QW Dosing Group 1 administered 350 mg/kg/day (Group 3QW1-2) was 2100 mg/kg.

On Days 4, 8, 11 and 15, relative tumor volume (RTV) was calculated as an index of anti-tumor effect during the dosing period for each group (Fig. 5).

As shown in Fig. 5, both the Continuous Dosing Group and 3QW Dosing Group 1 showed the effect of reducing tumor volume compared to the Control Group on Day 15. When comparing Group C-1 and Group 3QW1-1 with the total dosage of 1400 mg/ kg, the effect of reducing tumor volume in Group 3QW1-1 was similar as Group C-1, and the difference is not statistically significant. When comparing Group C-2 and Group 3QW1-2 with the total dosage of 2100 mg/kg, the effect of reducing tumor volume in Group 3QW1-2 and Group C-2 was similar, and the difference is not statistically significant.

On the other hand, as shown in Fig. 6, body weight loss was observed in the Continuous Dosing Group and 3QW Dosing Group 1. When comparing Group C-1 and Group 3QW1-1 with the total dosage of 1400 mg/kg, the recovery indicated by the BW change % values in Group 3QW1-1 on Day 15 was better with a statistically significant difference relative to Group C-1 (t-test, $p<0.05$). Similarly, when comparing Group C-2 and Group 3QW1-2 with the total dosage of 2100 mg/kg, the recovery indicated by the BW change % values in Group 3QW1-2 on Day 15 was better with a statistically significant difference relative to Group C-2 (t-test, $p<0.05$). This result indicates improved body weight reduction as an index of side-effects of Compound A administration in the intermittent dosing regimen (3QW Dosing Group 1) over the continuous daily dosing regimen (Continuous Dosing Group).

The above results revealed that the intermittent regimen (dosing three days consecutively per week) is a less toxic dosing schedule with comparable anti-tumor effect relative to a conventional continuous dosing schedule, and a very useful method that exhibits anti-tumor effects while avoiding toxicity resulting from Compound A administration.

**Example 4**

Anti-tumor Effect and body weight change in 3QW Dosing Regimen 2

Similar to Example 1, anti-tumor effect and body weight change were studied in mice harboring MV-4-11 cells among the following five groups: Control group (○), Continuous Dosing Group (●: 100 mg/kg/day; ◊: 150 mg/kg/day) and 3QW Dosing Group 2 (▲: 233 mg/kg/day; ♦: 350 mg/kg/day).
5-chloro-2-(N-((1S,2R)-2-(6-fluoro-2,3-dimetylphenyl)-1-(5-oxo-4,5-dihydro-1,3,4-o xadiazol-2-yl)propyl)sulfamoyl)benzamide (Compound A) was suspended in 5 mg/mL hypromellose solution to provide appropriate test samples for 100 mg/kg, 150 mg/kg, 233 mg/kg, and 350 mg/kg dosing regimen. Animals of the Control group were not treated.
To mice in the Continuous Dosing Group, 100 mg/kg/day or 150mg/kg/day of Compound A was orally administered once daily from Day 1 to Day 14. The total dosage for mice in the Continuous Dosing Group administered 100 mg/kg/day (Group C-1) was 1400 mg/kg, and the total dosage for mice in the Continuous Dosing Group administered 150 mg/kg/day (Group C-2) was 2100 mg/kg.
To mice in the 3QW Dosing Group 2, 233 mg/kg/day or 350 mg/kg/day of Compound A was orally administered every other day three times per week for two weeks (Days 1, 3, 5, 8, 10 and 12). The total dosage for mice in the 3QW Dosing Group 2 administered 233 mg/kg/day (Group 3QW2-1) was 1400 mg/kg, and the total dosage for mice in the 3QW Dosing Group 2 administered 350 mg/kg/day (Group 3QW2-2) was 2100 mg/kg.
On Days 4, 8, 11 and 15, relative tumor volume (RTV) was calculated as an index of anti-tumor effect during the dosing period for each group (Fig. 7).
As shown in Fig. 7, both Continuous Dosing Group and 3QW Dosing Group 2 showed the effect of reducing tumor volume compared to the Control Group on Day 15. When comparing Group C-1 and Group 3QW2-1 with the total dosage of 1400 mg/ kg, the effect of reducing tumor volume in Group 3QW2-1 was higher than Group C-1, and the difference is statistically significant (t-test, $P<0.05$). When comparing Group C-2 and Group 3QW2-2 with the total dosage of 2100 mg/kg, the effect of reducing tumor volume in Group QOD-2 and Group C-2 was similar, and the difference is not statistically significant.
On the other hand, as shown in Fig. 8, body weight loss was observed in the Continuous Dosing Group and 3QW Dosing Group 2. When comparing Group C-1 and Group 3QW2-1 with the total dosage of 1400 mg/kg, the recovery indicated by the BW change % values in Group 3QW2-1 on Day 15 was better with a statistically significant difference relative to Group C-1 (t-test, $p<0.05$). Similarly, when comparing Group C-2 and Group 3QW2-2 with the total dosage of 2100 mg/kg, the recovery indicated by the BW change % values in Group 3QW2-2 on Day 15 was better with a statistically significant difference relative to Group C-2 (t-test, $p<0.05$). This result indicates improved body weight reduction as an index of side-effects of Compound A administration in the intermittent dosing regimen (3QW Dosing Group 2) over the continuous daily dosing regimen (Continuous Dosing Group).
The above results revealed that the intermittent regimen (dosing every other day three times per week) is a less toxic dosing schedule with higher or comparable anti-tumor effect relative to a conventional continuous dosing schedule, and a very useful method that exhibits higher or comparable anti-tumor effects while avoiding toxicity resulting from Compound A administration.

**Example 5**

Anti-tumor Effect and body weight change in 5QW Dosing Regimen 1

Similar to Example 1, anti-tumor effect and body weight change were studied in mice harboring MV-4-11 cells among the following five groups: Control group (○), Continuous Dosing Group (●: 100 mg/kg/day; ◊: 150 mg/kg/day) and 5QW Dosing Group 1 (♦: 140 mg/kg/day; □: 210 mg/kg/day).
5-chloro-2-(N-((1S,2R)-2-(6-fluoro-2,3-dimetylphenyl)-1-(5-oxo-4,5-dihydro-1,3,4-o xadiazol-2-yl)propyl)sulfamoyl)benzamide (Compound A) was suspended in 5 mg/mL hypromellose solution to provide appropriate test samples for 100 mg/kg, 150 mg/kg, 140 mg/kg, and 210 mg/kg dosing regimen. Animals of the Control group were not treated.
To mice in the Continuous Dosing Group, 100 mg/kg/day or 150mg/kg/day of Compound A was orally administered once daily from Day 1 to Day 14. The total dosage for mice in the Continuous Dosing Group administered 100 mg/kg/day (Group C-1) was 1400 mg/kg, and the total dosage for mice in the Continuous Dosing Group administered 150 mg/kg/day (Group C-2) was 2100 mg/kg.
To mice in 5QW Dosing Group 1, 140 mg/kg/day or 210 mg/kg/day of Compound A was orally administered five days consecutively per week for two weeks (Days 1-5 and 8-12). The total dosage for mice in the 5QW Dosing Group 1 administered 140 mg/ kg/day (Group 5QW1-1) was 1400 mg/kg, and the total dosage for mice in the 5QW Dosing Group 1 administered 210 mg/kg/day (Group 5QW1-2) was 2100 mg/kg.
On Days 4, 8, 11 and 15, relative tumor volume (RTV) was calculated as an index of anti-tumor effect during the dosing period for each group (Fig. 9).
As shown in Fig. 9, both the Continuous Dosing Group and 5QW Dosing Group 1 showed the effect of reducing

tumor volume compared to the Control Group on Day 15. When comparing Group C-1 and Group 5QW1-1 with the total dosage of 1400 mg/ kg, the effect of reducing tumor volume in Group 5QW1-1 was similar to Group C-1, and the difference is not statistically significant. When comparing Group C-2 and Group 5QW1-2 with the total dosage of 2100 mg/kg, the effect of reducing tumor volume in Group 5QW1-2 and Group C-2 was similar, and the difference is not statistically significant.

On the other hand, as shown in Fig. 10, body weight loss was observed in the Continuous Dosing Group and 5QW Dosing Group 1. When comparing Group C-1 and Group 5QW1-1 with the total dosage of 1400 mg/kg, the recovery indicated by the BW change % values in Group 5QW1-1 on Day 15 was better with a statistically significant difference relative to Group C-1 (t-test, $p<0.05$). Similarly, when comparing Group C-2 and Group 5QW1-2 with the total dosage of 2100 mg/kg, the recovery indicated by the BW change % values in Group 5QW1-2 on Day 15 was better with a statistically significant difference relative to Group C-2 (t-test, $p<0.05$). This result indicates improved body weight reduction as an index of side-effects of Compound A administration in the intermittent dosing regimen (5QW Dosing Group 1) over the continuous daily dosing regimen (Continuous Dosing Group).

The above results revealed that the intermittent regimen (dosing five days consecutively per week) is a less toxic dosing schedule with comparable anti-tumor effect relative to a conventional continuous dosing schedule, and a very useful method that exhibits anti-tumor effects while avoiding toxicity resulting from Compound A administration.

**Example 6**

Anti-tumor Effect and body weight change in 5QW Dosing Regimen 2

Similar to Example 1, anti-tumor effect and body weight change were studied in mice harboring MV-4-11 cells among the following five groups: Control group (○), Continuous Dosing Group (●: 100 mg/kg/day; ◊: 150 mg/kg/day) and 5QW Dosing Group 2 (■: 140 mg/kg/day; x: 210 mg/kg/day).

5-chloro-2-(N-((1S,2R)-2-(6-fluoro-2,3-dimetylphenyl)-1-(5-oxo-4,5-dihydro-1,3,4-o xadiazol-2-yl)propyl)sulfamoyl)benzamide (Compound A) was suspended in 5 mg/mL hypromellose solution to provide appropriate test samples for 100 mg/kg, 150 mg/kg, 140 mg/kg, and 210 mg/kg dosing regimen. Animals of the Control group were not treated.

To mice in the Continuous Dosing Group, 100 mg/kg/day or 150mg/kg/day of Compound A was orally administered once daily from Day 1 to Day 14. The total dosage for mice in the Continuous Dosing Group administered 100 mg/kg/day (Group C-1) was 1400 mg/kg, and the total dosage for mice in the Continuous Dosing Group administered 150 mg/kg/day (Group C-2) was 2100 mg/kg.

To mice in 5QW Dosing Group 2, 140 mg/kg/day or 210 mg/kg/day of Compound A was orally administered five days per week for two weeks (Days 1-2, 4-6, 8-9, an 11-13). The total dosage for mice in the 5QW Dosing Group 2 administered 140 mg/ kg/day (Group 5QW2-1) was 1400 mg/kg, and the total dosage for mice in the 5QW Dosing Group 2 administered 210 mg/kg/day (Group 5QW2-2) was 2100 mg/kg.

On Days 4, 8, 11 and 15, relative tumor volume (RTV) was calculated as an index of anti-tumor effect during the dosing period for each group (Fig. 11).

As shown in Fig. 11, both the Continuous Dosing Group and 5QW Dosing Group 2 showed the effect of reducing tumor volume compared to the Control Group on Day 15. When comparing Group C-1 and Group 5QW2-1 with the total dosage of 1400 mg/ kg, the effect of reducing tumor volume in Group 5QW2-1 was similar to Group C-1, and the difference is not statistically significant. When comparing Group C-2 and Group 5QW2-2 with the total dosage of 2100 mg/kg, the effect of reducing tumor volume in Group 5QW2-2 was higher than Group C-2 with a statistically significant difference (t-test, $p<0.05$).

On the other hand, as shown in Fig 12, body weight loss was observed in the Continuous Dosing Group and 5QW Dosing Group 1. When comparing BW change % values of Group C-1 and Group 5QW2-1 with the total dosage of 1400 mg/kg on Day 15, a statistically significant difference could not be observed. However, when comparing Group C-2 and Group 5QW2-2 with the total dosage of 2100 mg/kg, the recovery indicated by the BW change % values in Group 5QW2-2 on Day 15 was better with a statistically significant difference relative to Group C-2 (t-test, $p<0.05$). This result suggests improved body weight reduction as an index of side-effects of Compound A administration in the intermittent dosing regimen (5QW Dosing Group 2) over the continuous daily dosing regimen (Continuous Dosing Group).

Since the anti-tumor effects were the same among the 5QW Dosing Group 2 and the Continuous Dosing Group, or higher in the former Group compared to the latter, the above results revealed that the intermittent regimen (dosing five days per week) is a less toxic dosing schedule with higher or comparable anti-tumor effect than conventional continuous dosing schedules, and a very useful method that exhibits anti-tumor effects while avoiding toxicity resulting from Compound A administration.

All animal experimental protocols of these studies were judged by the Institutional Animal Care and Use Committee and approved by the Director of Institution based on "Guidelines for Animal Experiment of Taiho Pharmaceutical Co., Ltd.". The handling of animals was performed appropriately in accordance with those guidelines.

**Example 7**

Correlation of SLFN11 expression with the cytotoxic effects of Compound A

Purpose:

Whether SLFN11 expression correlates with the cytotoxic effects of Compound A or not was examined.

Methods:

Cell lines

The RD-ES, SK-ES-1, SK-LMS-1, SJSA-1, SW1353, U-2OS, NCI-H460 and CFPAC-1 cell lines were obtained from the ATCC. The A673 cell line was obtained from DS Pharma Biomedical Co., Ltd. The ESS-1 cell line was obtained from DSMZ. The MG-63, HOS and G-292 clone A141B1 cell lines were obtained from Dainippon Pharmaceutical Co., Ltd.

Cell proliferation assay

Cell proliferation assay was performed in accordance with conventional methods, available in Hasako, S., et al. Mol Cancer Ther 17, 1648-1658 (2018). Briefly, cells were plated and incubated for 1 day, followed by 3 days of exposure to the compound. After exposure to Compound A, viable cells were detected by using the CellTiter-Glo luminescent cell viability assay (Promega). The experiment was performed in triplicates.

Correlation analysis

Correlation analysis of SLFN11 expression and Compound A cytotoxicity was performed using Pearson's correlation coefficient ($P < 0.05$) in JMP 13 software. The gene expression data of cell lines were available online CCLE (http://www.broadinstitute.org/ccle/home). Compound A cytotoxicity data from 11 cell lines in Table 1 were used for correlation analysis (data for correlation was not shown).

[Table 1]

| Cell line | Origin |
|---|---|
| RD-ES | Ewing sarcoma |
| A673 | Ewing sarcoma |
| SK-ES-1 | Ewing sarcoma |
| ESS-1 | endometrial stromal sarcoma |
| MG-63 | osteosarcoma |
| SK-LMS-1 | vulvar leiomyosarcoma |
| SJSA-1 | osteosarcoma |
| SW1353 | chondrosarcoma |
| HOS | osteosarcoma |
| G-292 clone A141B1 | osteosarcoma |
| U-2OS | osteosarcoma |

siRNA treatment

A673 cells, NCI-H460 cells and CFPAC-1 cells were transfected with 1 nM siRNAs for 24 h using Lipofectamine(R)RNAiMAX Reagent (Thermo Fisher Scientific Inc.) and then re-seeded onto a 96-well plate for cell proliferation assay and caspase induction assay and onto dishes for immunoblotting analysis. siRNAs were obtained from Thermo Fisher Scientific Inc. as follows: Silencer(R) Select Negative Control #1 siRNA (#4390843 used as siControl), SLFN11 Silencer(R) Select Pre-designed siRNA (#s40703 used as siSLFN11-1, #s40704 as siSLFN11-2, and #s40702 as siSLFN11-3).

The detection of SLFN11 expression was performed by immunoblotting analysis in accordance with conventional methods, available in Hasako, et al. ibid. Briefly the cells were collected and whole cell protein was extracted on ice with M-PER (Thermo Fisher Scientific, Inc.). Proteins were separated by SDS-PAGE and analyzed by Western blotting. SLFN11 was detected by anti-SLFN11 polyclonal antibody (#HPA023030, Atlas Antibodies).

Cell proliferation assay was performed in accordance with conventional methods, available in Hasako, et al., ibid. Briefly, cells treated with siRNAs for 24 h were plated onto 96 well plate and incubated for 24 h, followed by 3 days of exposure to the compound A. After compound exposure, viable cells were detected by using the CellTiter-Glo luminescent cell viability assay (Promega).

Caspase induction assay was performed in accordance with conventional methods, available in Hasako., et al., ibid. Briefly, the cells with siRNAs for 24 h were seeded onto 96 well plate, incubated for 24 h, and then treated with Compound A for 24 h. Caspase induction was detected by using the Caspase-Glo 3/7 assay (Promega).

Results:

The correlation between SLFN11 mRNA expression and the relative growth percent of cells at 100 micro mol/L treatment by Compound A was analyzed in 11 sarcoma cell lines listed in Table 1.

In this analysis, a significant correlation between SLFN11 mRNA expression and relative cell growth (%) at 100 micro mol/L Compound A was detected (Fig. 13).

To determine whether SLFN11 directly affects the cytotoxic property of Compound A, the effect of SLFN11 knockdown with siRNA on the cytotoxicity of Compound A in an Ewing sarcoma cell line A673 was examined. As a result, SLFN11 knockdown significantly suppressed the cytotoxic effect of Compound A (Fig. 14).

Furthermore, the suppression of Compound A (10 micro mol/L)-induced caspase-3/7 activation in A673 cells transfected with siRNA against SLFN11 was observed (Fig. 15). Further, reduction of SLFN11 expression in the A673 cells transfected with siRNA against SLFN11 was confirmed (Fig. 16).

To further determine whether SLFN11 directly affects the cytotoxic property of Compound A in the other cancer types, the effects of SLFN11 knockdown with siRNA on the cytotoxicity of Compound A in a non-small cell lung cancer cell line NCI-H460 and a pancreatic cancer cell line CFPAC-1 were examined. As a result, SLFN11 knockdown significantly suppressed the cytotoxic effect of Compound A (Fig. 17 and 18).

Furthermore, the suppression of Compound A (10 micro mol/L)-induced caspase-3/7 activation in NCI-H460 cells and CHPAC-1 cells, both of which had been transfected with siRNA against SLFN11, were observed (Fig. 19).

## Claims

**1.** A compound for use in preventing and/or treating a tumor in a patient in need thereof, wherein the compound is 5-chloro-2-(N-((1S,2R)-2-(6-fluoro-2,3-dimetylphenyl)-1-(5-oxo-4,5-dihydro-1,3,4-oxadiazol-2-yl)propyl)sulfamoyl) benzamide or a salt thereof, by administering an effective amount of the compound to the patient on an intermittent administration schedule for two weeks comprising dosing 1 to 5 days per week, wherein the administration schedule is selected from

(a) an administration schedule comprising dosing one day per week;
(b) an administration schedule comprising three dosing days and four non-dosing days per week, preferably an administration schedule comprising

(b1) dosing for three consecutive days followed by four non-dosing days in one week or
(b2) dosing every other day three times followed by two non-dosing days in one week; or

(c) an administration schedule comprising five dosing days and two non-dosing days per week, preferably an administration schedule comprising

(c1) dosing for five consecutive days followed by two non-dosing days in one week, or
(c2) (a) two consecutive dosing days and one drug holiday, followed by (b) three consecutive dosing days and one drug holiday, or comprising (b) three consecutive dosing days and one non-dosing day, followed by (a) two consecutive dosing days and one non-dosing day, in one week; or

(d) administration schedule comprises dosing every other day and a total of seven days within two weeks.

**2.** The compound for use of claim 1, wherein the administration schedule is based on a 4-week cycle, and the cycle is

performed once or repeated two or more times.

3. The compound for use of claim 1, wherein 5-chloro-2-(N-((1S,2R)-2-(6-fluoro-2,3-dimetylphenyl)-1-(5-oxo-4,5-dihydro-1,3,4-oxadiazol-2-yl)propyl)sulfamoyl)benzamide or a salt thereof is administered orally.

4. The compound for use of claim 1, wherein an RNR-related disease is acute myeloid leukemia (AML), including relapsed or refractory (R/R) acute myeloid leukemia (AML).

5. The compound for use of claim 1, wherein an RNR-related disease is a SLFN11-positive tumor.

6. A compound for use in treating acute myeloid leukemia (AML) in a patient in need thereof, wherein the compound is 5-chloro-2-(N-((1S,2R)-2-(6-fluoro-2,3-dimetylphenyl)-1-(5-oxo-4,5-dihydro-1,3,4-oxadiazol-2-yl)propyl)sulfamoyl) benzamide or a salt thereof, by administering an effective amount of the compound to the patient on an intermittent administration schedule for two weeks comprising dosing 1 to 5 days per week.

7. The compound for use of claim 6, wherein 5-chloro-2-(N-((1S,2R)-2-(6-fluoro-2,3-dimetylphenyl)-1-(5-oxo-4,5-dihydro-1,3,4-oxadiazol-2-yl)propyl)sulfamoyl)benzamide or a salt thereof is administered as a single agent.

8. The compound for use of claim 6, wherein 5-chloro-2-(N-((1S,2R)-2-(6-fluoro-2,3-dimetylphenyl)-1-(5-oxo-4,5-dihydro-1,3,4-oxadiazol-2-yl)propyl)sulfamoyl)benzamide or a salt thereof is administered with one or more additional anti-tumor agents.

9. A compound for use in reducing the risk of recurrence of acute myeloid leukemia (AML) or death for a patient diagnosed with AML, wherein the compound is 5-chloro-2-(N-((1S,2R)-2-(6-fluoro-2,3-dimetylphenyl)-1-(5-oxo-4,5-dihydro-1,3,4-oxadiazol-2-yl)propyl)sulfamoyl)benzamide or a salt thereof, by administering an effective amount of the compound to the patient on an intermittent administration schedule for two weeks comprising dosing 1 to 5 days per week.

10. The compound for use of claim 9, wherein 5-chloro-2-(N-((1S,2R)-2-(6-fluoro-2,3-dimetylphenyl)-1-(5-oxo-4,5-dihydro-1,3,4-oxadiazol-2-yl)propyl)sulfamoyl)benzamide or a salt thereof is administered as a single agent.

11. The compound for use of claim 9, wherein 5-chloro-2-(N-((1S,2R)-2-(6-fluoro-2,3-dimetylphenyl)-1-(5-oxo-4,5-dihydro-1,3,4-oxadiazol-2-yl)propyl)sulfamoyl)benzamide or a salt thereof is administered with one or more additional anti-tumor agents.

## Patentansprüche

1. Eine Verbindung zur Vorbeugung und/oder Behandlung eines Tumors bei einem Patienten, der diese benötigt, wobei es sich bei der Verbindung um 5-Chlor-2-(N-((1S,2R)-2-(6-Fluor-2,3-dimethylphenyl)-1-(5-oxo-4,5-dihydro-1,3,4-oxadiazol-2-yl)propyl)sulfamoyl)benzamid oder ein Salz davon handelt, durch Verabreichung einer wirksamen Menge der Verbindung an den Patienten über einen Zeitraum von zwei Wochen mit einer intermittierenden Dosierung an 1 bis 5 Tagen pro Woche, wobei das Dosierungsschema wie folgt ausgewählt ist:

   (a) ein Dosierungsschema mit einem Tag pro Woche;
   (b) ein Dosierungsschema mit drei Dosierungstagen und vier nicht-dosierenden Tagen pro Woche, vorzugsweise ein Dosierungsschema mit:

      (b1) drei aufeinanderfolgenden Dosierungstagen, gefolgt von vier nicht-dosierenden Tagen innerhalb einer Woche oder
      (b2) dreimaliger Dosierung jeden zweiten Tag, gefolgt von zwei nicht-dosierenden Tagen innerhalb einer Woche. oder

   c) ein Verabreichungsschema mit fünf Behandlungstagen und zwei Behandlungsfreien Tagen pro Woche, vorzugsweise ein Verabreichungsschema, das Folgendes umfasst:

      c1) Behandlung an fünf aufeinanderfolgenden Tagen, gefolgt von zwei Behandlungsfreien Tagen innerhalb einer Woche, oder

c2) (a) zwei aufeinanderfolgende Behandlungstage und eine Behandlungspause, gefolgt von (b) drei aufeinanderfolgenden Behandlungstagen und einer Behandlungspause, oder
(b) drei aufeinanderfolgende Behandlungstage und ein Behandlungsfreier Tag, gefolgt von (a) zwei aufeinanderfolgenden Behandlungstagen und einem Behandlungsfreier Tag innerhalb einer Woche; oder

d) ein Verabreichungsschema, das eine Behandlung jeden zweiten Tag und insgesamt sieben Tage innerhalb von zwei Wochen umfasst.

**2.** Die Verbindung zur Verwendung nach Anspruch 1, wobei das Verabreichungsschema auf einem 4-Wochen-Zyklus basiert und der Zyklus einmal oder zwei- oder mehrmals wiederholt wird.

**3.** Die Verbindung zur Verwendung nach Anspruch 1, wobei 5-Chlor-2-(N-((1S,2R)-2-(6-Fluor-2,3-dimethylphenyl)-1-(5-oxo-4,5-dihydro-1,3,4-oxadiazol-2-yl)propyl)sulfamoyl)benzamid oder ein Salz davon oral verabreicht wird.

**4.** Die Verbindung zur Verwendung nach Anspruch 1, wobei es sich bei der RNR-assoziierten Erkrankung um eine akute myeloische Leukämie (AML), einschließlich rezidivierter oder refraktärer (R/R) akuter myeloischer Leukämie (AML), handelt.

**5.** Die Verbindung zur Verwendung nach Anspruch 1, wobei es sich bei der RNR-assoziierten Erkrankung um einen SLFN11-positiven Tumor handelt.

**6.** Eine Verbindung zur Behandlung der akuten myeloischen Leukämie (AML) bei einem Patienten, der diese benötigt, wobei es sich bei der Verbindung um 5-Chlor-2-(N-((1S,2R)-2-(6-Fluor-2,3-dimethylphenyl)-1-(5-oxo-4,5-dihydro-1,3,4-oxadiazol-2-yl)propyl)sulfamoyl)benzamid oder ein Salz davon handelt, durch Verabreichung einer wirksamen Menge der Verbindung an den Patienten in einem intermittierenden Verabreichungsschema über zwei Wochen, bestehend aus einer Dosierung an 1 bis 5 Tagen pro Woche.

**7.** Die Verbindung nach Anspruch 6, wobei 5-Chlor-2-(N-((1S,2R)-2-(6-Fluor-2,3-dimethylphenyl)-1-(5-oxo-4,5-dihydro-1,3,4-oxadiazol-2-yl)propyl)sulfamoyl)benzamid oder ein Salz davon als Monotherapie verabreicht wird.

**8.** Die Verbindung nach Anspruch 6, wobei 5-Chlor-2-(N-((1S,2R)-2-(6-Fluor-2,3-dimethylphenyl)-1-(5-oxo-4,5-dihydro-1,3,4-oxadiazol-2-yl)propyl)sulfamoyl)benzamid oder ein Salz davon zusammen mit einem oder mehreren weiteren Antitumorwirkstoffen verabreicht wird.

**9.** Verbindung zur Verringerung des Risikos eines Wiederauftretens einer akuten myeloischen Leukämie (AML) oder des Todes bei einem Patienten mit AML, wobei es sich bei der Verbindung um 5-Chlor-2-(N-((1S,2R)-2-(6-Fluor-2,3-dimethylphenyl)-1-(5-oxo-4,5-dihydro-1,3,4-oxadiazol-2-yl)propyl)sulfamoyl)benzamid oder ein Salz davon handelt. Die Anwendung erfolgt durch intermittierende Verabreichung einer wirksamen Menge der Verbindung an den Patienten über einen Zeitraum von zwei Wochen, wobei die Gabe an 1 bis 5 Tagen pro Woche erfolgt.

**10.** Die Verbindung nach Anspruch 9, wobei 5-Chlor-2-(N-((1S,2R)-2-(6-Fluor-2,3-dimethylphenyl)-1-(5-oxo-4,5-dihydro-1,3,4-oxadiazol-2-yl)propyl)sulfamoyl)benzamid oder ein Salz davon als Monotherapie verabreicht wird.

**11.** Die Verbindung nach Anspruch 9, wobei 5-Chlor-2-(N-((1S,2R)-2-(6-Fluor-2,3-dimethylphenyl)-1-(5-oxo-4,5-dihydro-1,3,4-oxadiazol-2-yl)propyl)sulfamoyl)benzamid oder ein Salz davon zusammen mit einem oder mehreren weiteren Antitumorwirkstoffen verabreicht wird.

## Revendications

**1.** Composé pour utilisation dans la prévention et/ou le traitement d'une tumeur chez un patient qui en a besoin, dans lequel le composé est 5-chloro-2-(N-((1S,2R)-2-(6-fluoro-2,3-diméthylphényl)-1-(5-oxo-4,5-dihydro-1,3,4-oxadiazol-2-yl)propyl)sulfamoyl)benzamide ou un sel de celui-ci, en administrant une quantité efficace du composé au patient selon un calendrier d'administration intermittente pendant deux semaines comprenant une dose 1 à 5 jours par semaine, dans lequel le calendrier d'administration est sélectionné parmi

(a) un calendrier d'administration comprenant une dose un jour par semaine ;
(b) un calendrier d'administration comprenant trois jours avec dose et quatre jours sans dose par semaine, de

préférence un calendrier d'administration comprenant

(b1) une dose pendant trois jours consécutifs suivis de quatre jours sans dose sur une semaine ou
(b2) une dose tous les deux jours trois fois suivis de deux jours sans dose sur une semaine ; ou

(c) un calendrier d'administration comprenant cinq jours avec dose et deux jours sans dose par semaine, de préférence un calendrier d'administration comprenant

(c1) une dose pendant cinq jours consécutifs suivis de deux jours sans dose sur une semaine, ou
(c2) (a) deux jours avec dose consécutifs et une pause médicamenteuse, suivis de (b) trois jours avec dose consécutifs et une pause médicamenteuse, ou comprenant (b) trois jours avec dose consécutifs et un jour sans dose, suivis de (a) deux jours avec dose consécutifs et un jour sans dose, sur une semaine ; ou

(d) le calendrier d'administration comprend une dose tous les deux jours et un total de sept jours en deux semaines.

**2.** Composé pour une utilisation selon la revendication 1, dans lequel le calendrier d'administration est basé sur un cycle de 4 semaines, et le cycle est effectué une fois ou répété deux fois ou plus.

**3.** Composé pour utilisation selon la revendication 1, dans lequel 5-chloro-2-(N-((1S,2R)-2-(6-fluoro-2,3-diméthylphényl)-1-(5-oxo-4,5-dihydro-1,3,4-oxadiazol-2-yl)propyl)sulfamoyl)benzamide ou un sel de celui-ci est administré par voie orale.

**4.** Composé pour utilisation selon la revendication 1, dans lequel une maladie liée à une RNR est une leucémie myéloïde aiguë (LMA), y compris une leucémie myéloïde aiguë (LMA) en rechute ou réfractaire (R/R).

**5.** Composé pour utilisation selon la revendication 1, dans lequel une maladie liée à une RNR est une tumeur SLFN11 positive.

**6.** Composé pour utilisation dans le traitement d'une leucémie myéloïde aiguë (LMA) chez un patient qui en a besoin, dans lequel le composé est 5-chloro-2-(N-((1S,2R)-2-(6-fluoro-2,3-diméthylphényl)-1-(5-oxo-4,5-dihydro-1,3,4-oxadiazol-2-yl)propyl)sulfamoyl)benzamide ou un sel de celui-ci, en administrant une quantité efficace du composé au patient selon un calendrier d'administration intermittente pendant deux semaines comprenant une dose 1 à 5 jours par semaine.

**7.** Composé pour utilisation selon la revendication 6, dans lequel 5-chloro-2-(N-((1S,2R)-2-(6-fluoro-2,3-diméthylphényl)-1-(5-oxo-4,5-dihydro-1,3,4-oxadiazol-2-yl)propyl)sulfamoyl)benzamide ou un sel de celui-ci est administré sous forme d'un agent unique.

**8.** Composé pour utilisation selon la revendication 6, dans lequel 5-chloro-2-(N-((1S,2R)-2-(6-fluoro-2,3-diméthylphényl)-1-(5-oxo-4,5-dihydro-1,3,4-oxadiazol-2-yl)propyl)sulfamoyl)benzamide ou un sel de celui-ci est administré avec un ou plusieurs agents anti-tumoraux supplémentaires.

**9.** Composé pour utilisation dans la réduction du risque de rechute de leucémie myéloïde aiguë (LMA) ou de décès pour un patient diagnostiqué comme atteint d'une LMA, dans lequel le composé est 5-chloro-2-(N-((1S,2R)-2-(6-fluoro-2,3-diméthylphényl)-1-(5-oxo-4,5-dihydro-1,3,4-oxadiazol-2-yl)propyl)sulfamoyl)benzamide ou un sel de celui-ci, en administrant une quantité efficace du composé au patient selon un calendrier d'administration intermittente pendant deux semaines comprenant une dose 1 à 5 jours par semaine.

**10.** Composé pour utilisation selon la revendication 9, dans lequel 5-chloro-2-(N-((1S,2R)-2-(6-fluoro-2,3-diméthylphényl)-1-(5-oxo-4,5-dihydro-1,3,4-oxadiazol-2-yl)propyl)sulfamoyl)benzamide ou un sel de celui-ci est administré sous forme d'un agent unique.

**11.** Composé pour utilisation selon la revendication 9, dans lequel 5-chloro-2-(N-((1S,2R)-2-(6-fluoro-2,3-diméthylphényl)-1-(5-oxo-4,5-dihydro-1,3,4-oxadiazol-2-yl)propyl)sulfamoyl)benzamide ou un sel de celui-ci est administré avec un ou plusieurs agents anti-tumoraux supplémentaires.

[Fig. 1]

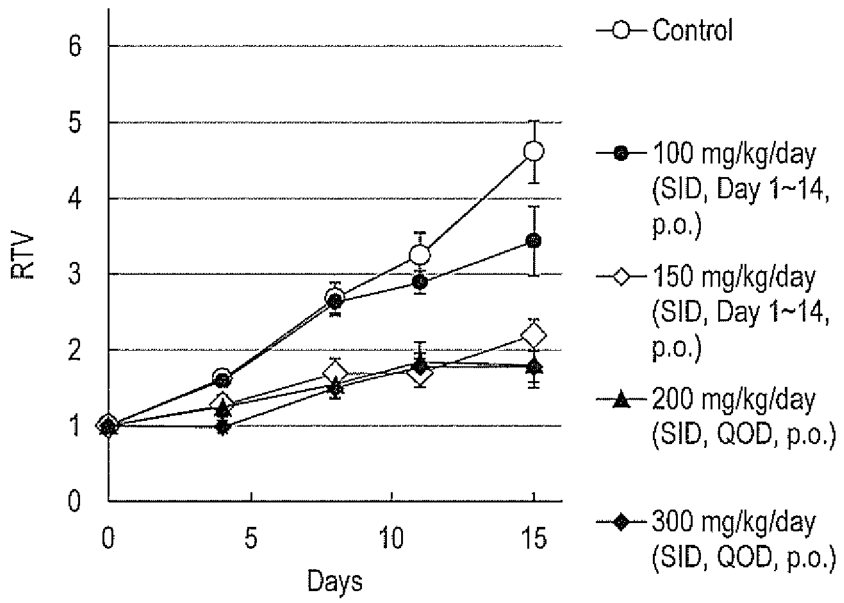
RTV
6
5
4
3
2
1
0
0
5
10
15
Days
Control
100 mg/kg/day (SID, Day 1~14, p.o.)
150 mg/kg/day (SID, Day 1~14, p.o.)
200 mg/kg/day (SID, QOD, p.o.)
300 mg/kg/day (SID, QOD, p.o.)

[Fig. 2]

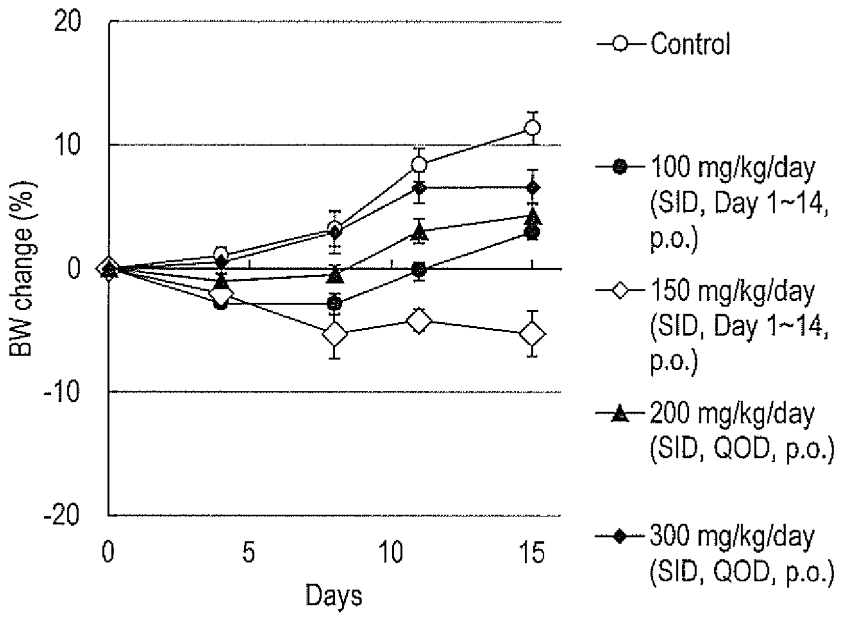
BW change (%)
20
10
0
-10
-20
0
5
10
15
Days
Control
100 mg/kg/day (SID, Day 1~14, p.o.)
150 mg/kg/day (SID, Day 1~14, p.o.)
200 mg/kg/day (SID, QOD, p.o.)
300 mg/kg/day (SID, QOD, p.o.)

[Fig. 3]

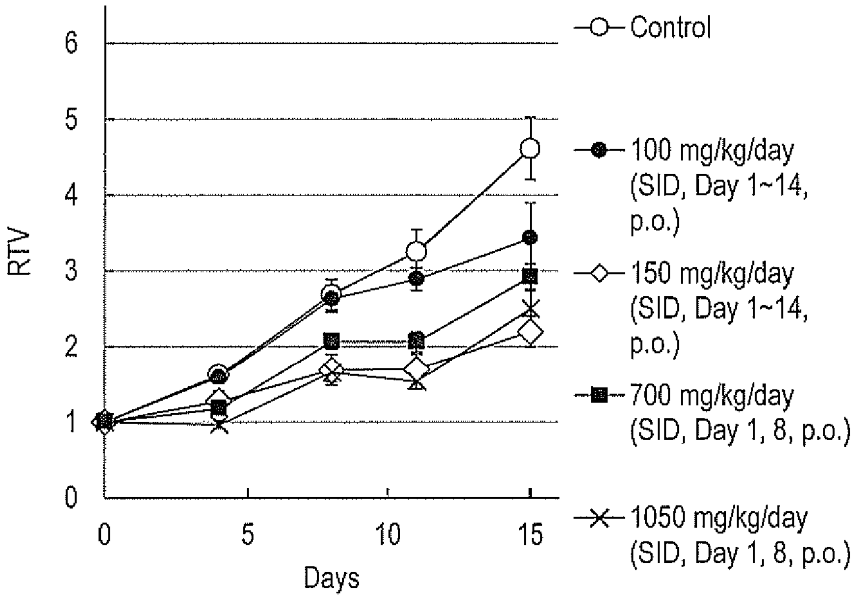
RTV
6
5
4
3
2
1
0
0
5
10
15
Days
Control
100 mg/kg/day (SID, Day 1~14, p.o.)
150 mg/kg/day (SID, Day 1~14, p.o.)
700 mg/kg/day (SID, Day 1, 8, p.o.)
1050 mg/kg/day (SID, Day 1, 8, p.o.)

[Fig. 4]

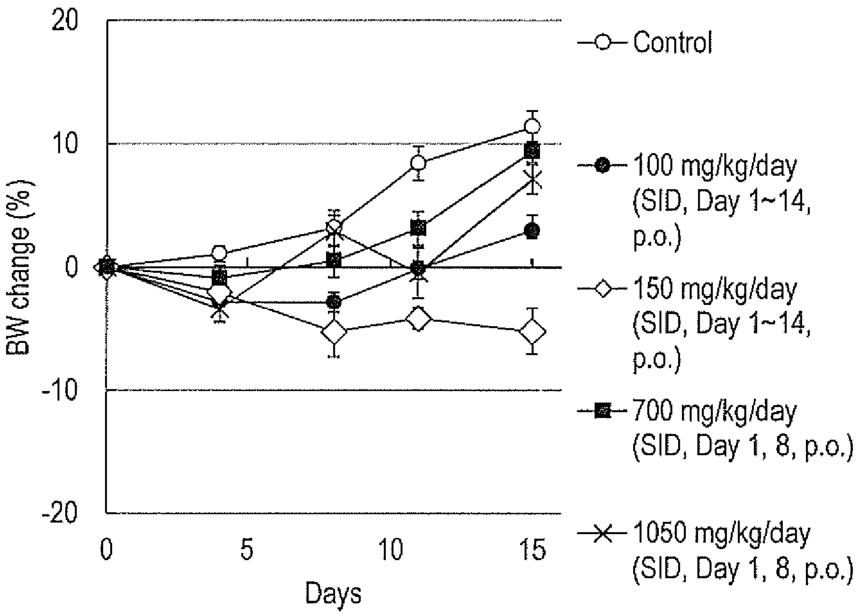
BW change (%)
20
10
0
-10
-20
0
5
10
15
Days
Control
100 mg/kg/day (SID, Day 1~14, p.o.)
150 mg/kg/day (SID, Day 1~14, p.o.)
700 mg/kg/day (SID, Day 1, 8, p.o.)
1050 mg/kg/day (SID, Day 1, 8, p.o.)

[Fig. 5]

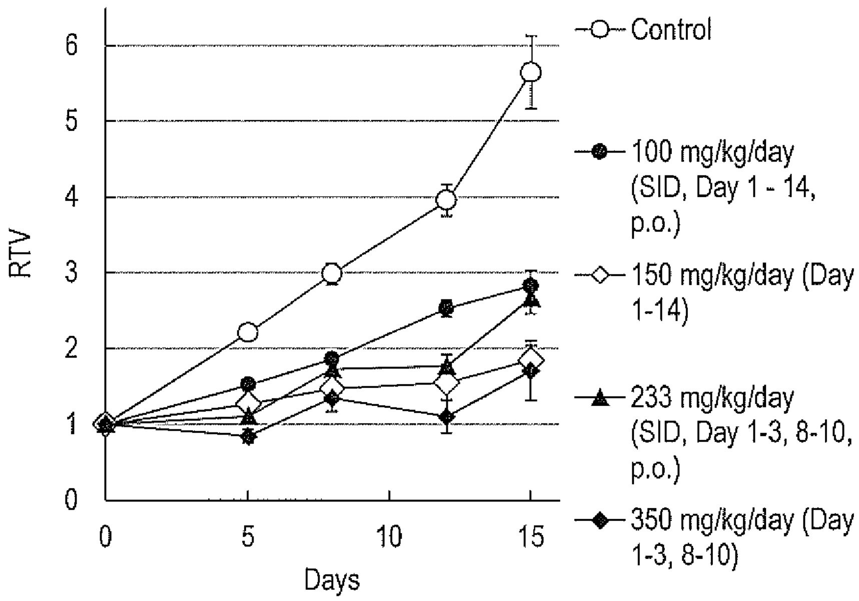

6
5
4
3
2
1
0
RTV
0
5
10
15
Days
Control
100 mg/kg/day (SID, Day 1 - 14, p.o.)
150 mg/kg/day (Day 1-14)
233 mg/kg/day (SID, Day 1-3, 8-10, p.o.)
350 mg/kg/day (Day 1-3, 8-10)


[Fig. 6]

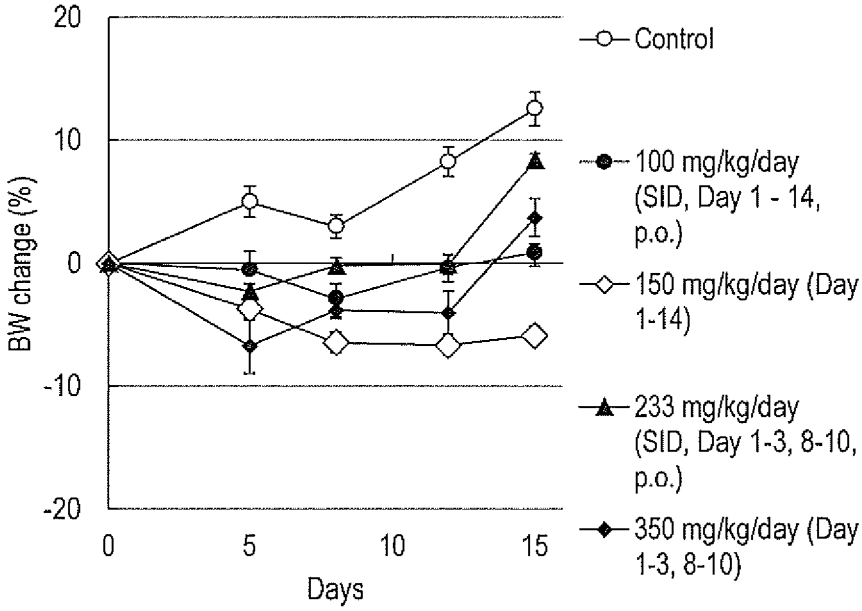

20
10
0
-10
-20
BW change (%)
0
5
10
15
Days
Control
100 mg/kg/day (SID, Day 1 - 14, p.o.)
150 mg/kg/day (Day 1-14)
233 mg/kg/day (SID, Day 1-3, 8-10, p.o.)
350 mg/kg/day (Day 1-3, 8-10)

[Fig. 7]

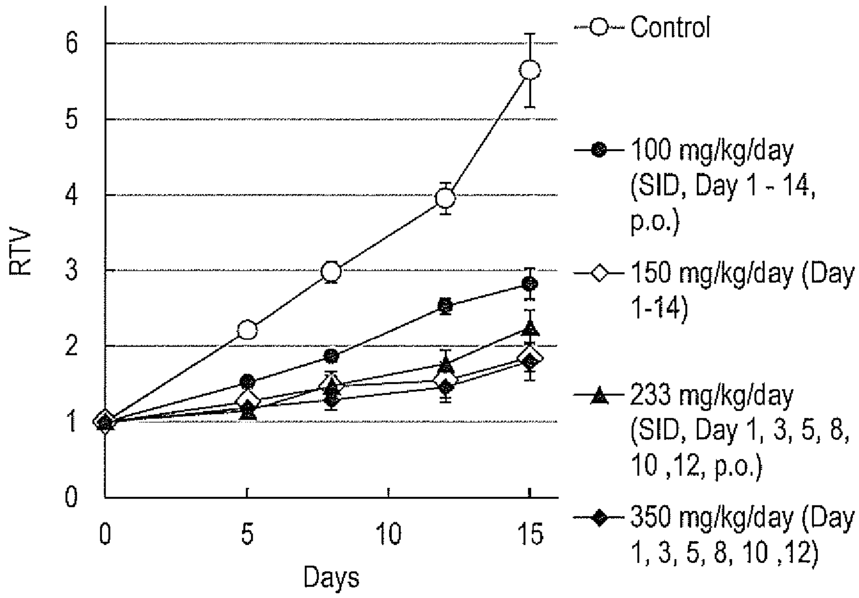
6
5
4
3
2
1
0
RTV
0
5
10
15
Days
Control
100 mg/kg/day (SID, Day 1 - 14, p.o.)
150 mg/kg/day (Day 1-14)
233 mg/kg/day (SID, Day 1, 3, 5, 8, 10 ,12, p.o.)
350 mg/kg/day (Day 1, 3, 5, 8, 10 ,12)

[Fig. 8]

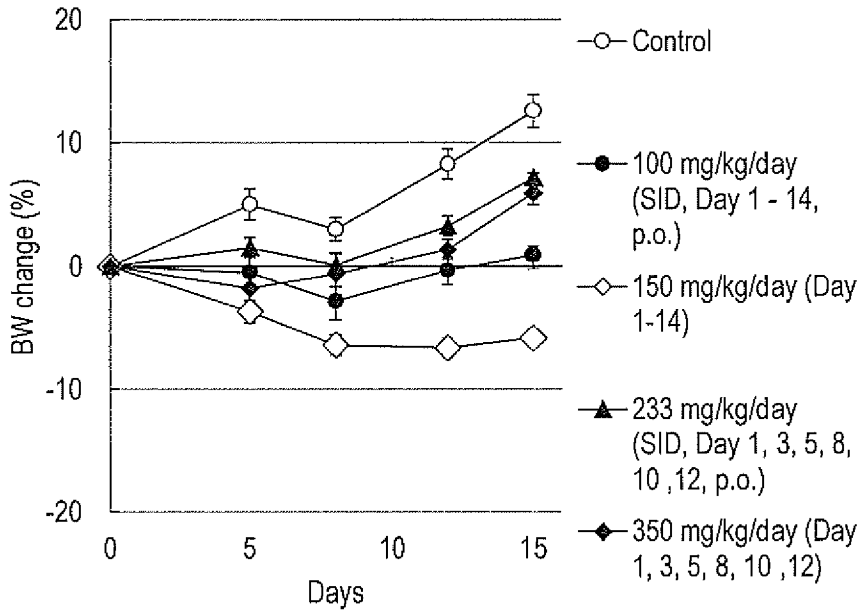
20
10
0
-10
-20
BW change (%)
0
5
10
15
Days
Control
100 mg/kg/day (SID, Day 1 - 14, p.o.)
150 mg/kg/day (Day 1-14)
233 mg/kg/day (SID, Day 1, 3, 5, 8, 10 ,12, p.o.)
350 mg/kg/day (Day 1, 3, 5, 8, 10 ,12)

[Fig. 9]

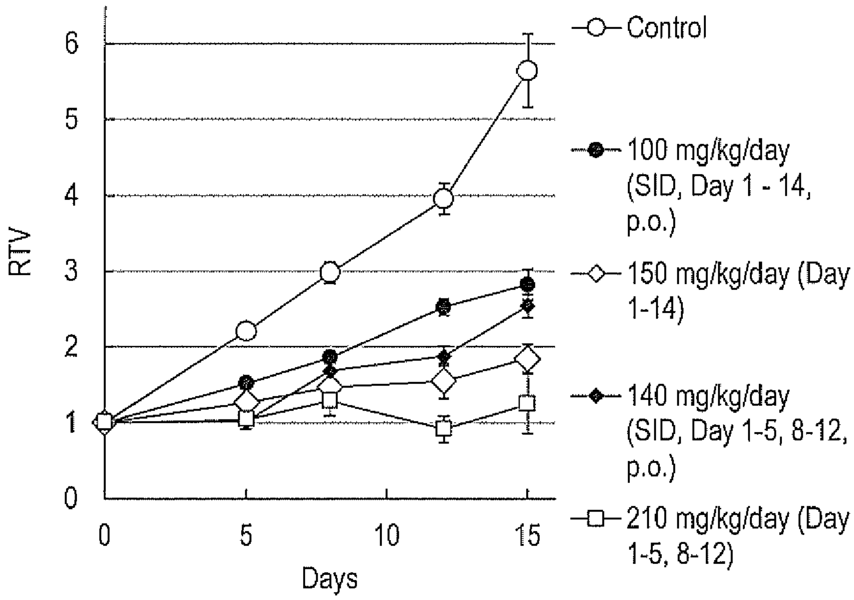
RTV
0
1
2
3
4
5
6
0
5
10
15
Days
Control
100 mg/kg/day (SID, Day 1 - 14, p.o.)
150 mg/kg/day (Day 1-14)
140 mg/kg/day (SID, Day 1-5, 8-12, p.o.)
210 mg/kg/day (Day 1-5, 8-12)

[Fig. 10]

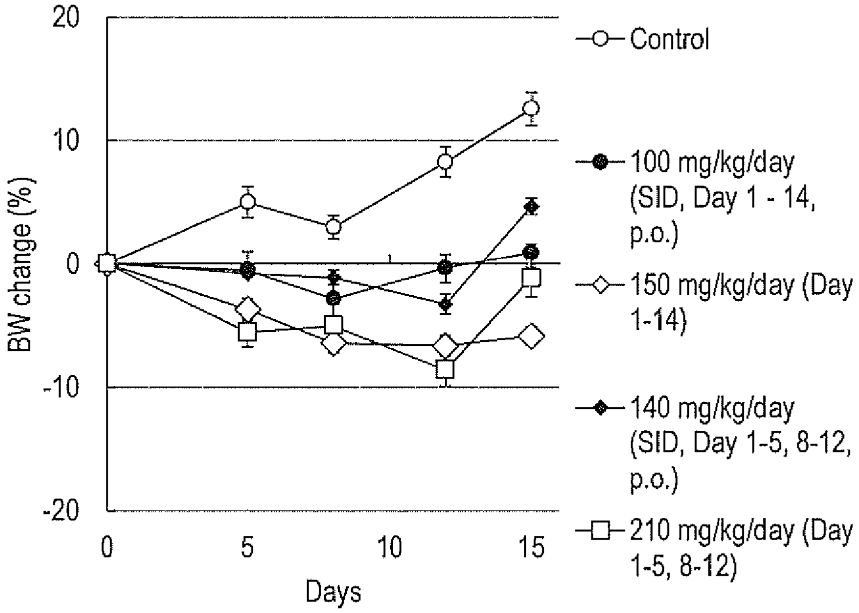
BW change (%)
20
10
0
-10
-20
0
5
10
15
Days
Control
100 mg/kg/day (SID, Day 1 - 14, p.o.)
150 mg/kg/day (Day 1-14)
140 mg/kg/day (SID, Day 1-5, 8-12, p.o.)
210 mg/kg/day (Day 1-5, 8-12)

[Fig. 11]

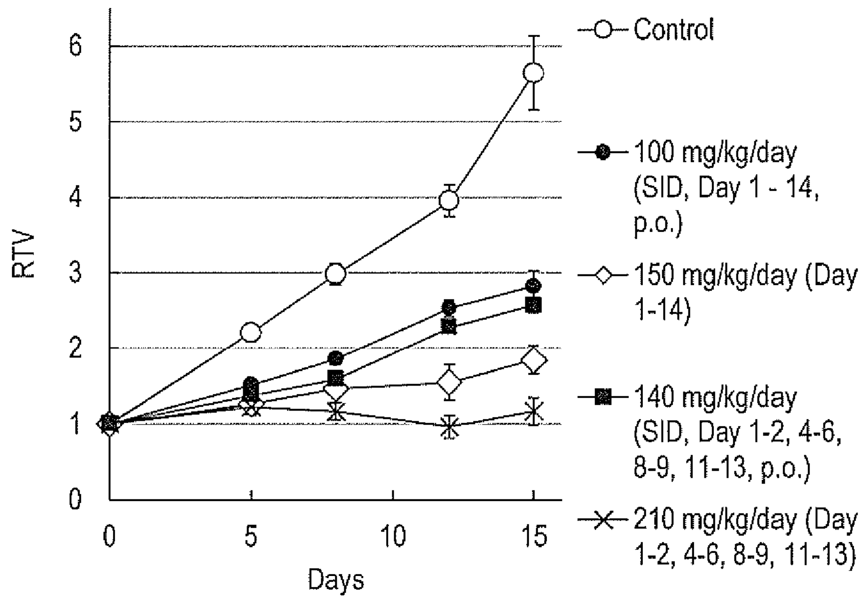
6
5
4
3
2
1
0
RTV
0
5
10
15
Days
Control
100 mg/kg/day (SID, Day 1 - 14, p.o.)
150 mg/kg/day (Day 1-14)
140 mg/kg/day (SID, Day 1-2, 4-6, 8-9, 11-13, p.o.)
210 mg/kg/day (Day 1-2, 4-6, 8-9, 11-13)

[Fig. 12]

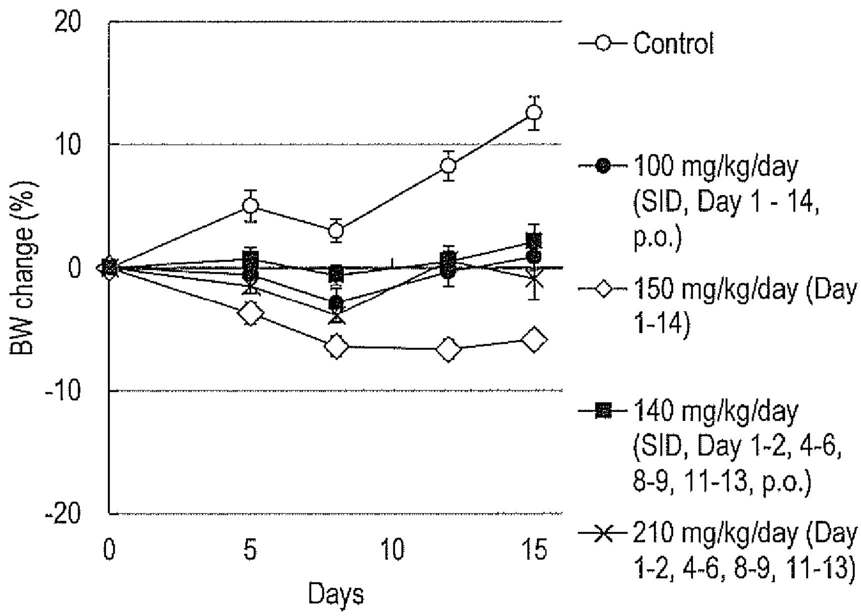
20
10
0
-10
-20
BW change (%)
0
5
10
15
Days
Control
100 mg/kg/day (SID, Day 1 - 14, p.o.)
150 mg/kg/day (Day 1-14)
140 mg/kg/day (SID, Day 1-2, 4-6, 8-9, 11-13, p.o.)
210 mg/kg/day (Day 1-2, 4-6, 8-9, 11-13)

[Fig. 13]

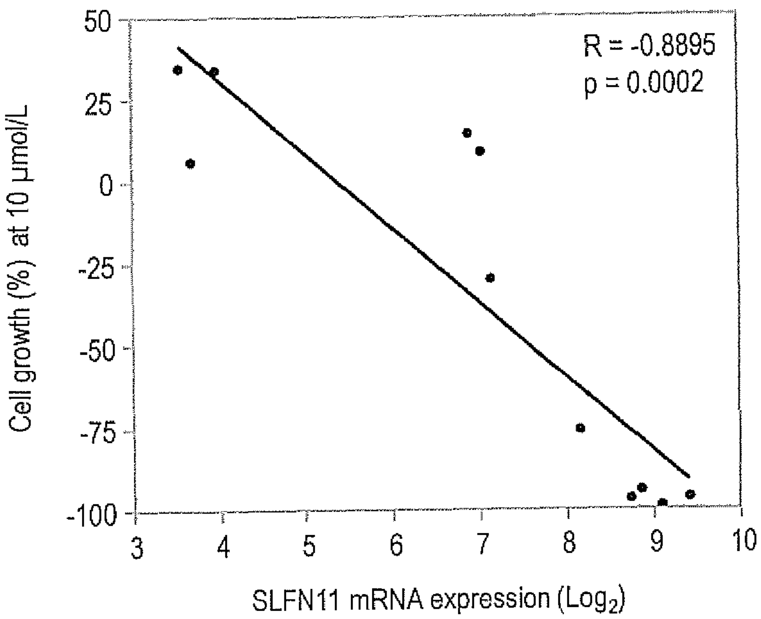
R = -0.8895
p = 0.0002
Cell growth (%) at 10 µmol/L
50
25
0
-25
-50
-75
-100
3
4
5
6
7
8
9
10
SLFN11 mRNA expression (Log2)

[Fig. 14]

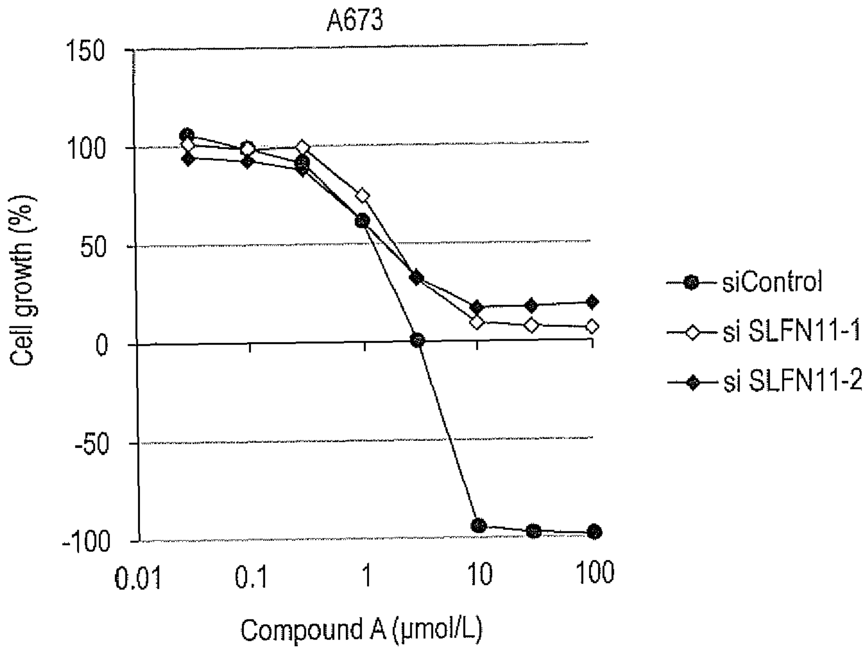
A673
150
100
50
0
-50
-100
Cell growth (%)
0.01
0.1
1
10
100
Compound A (µmol/L)
siControl
si SLFN11-1
si SLFN11-2

[Fig. 15]

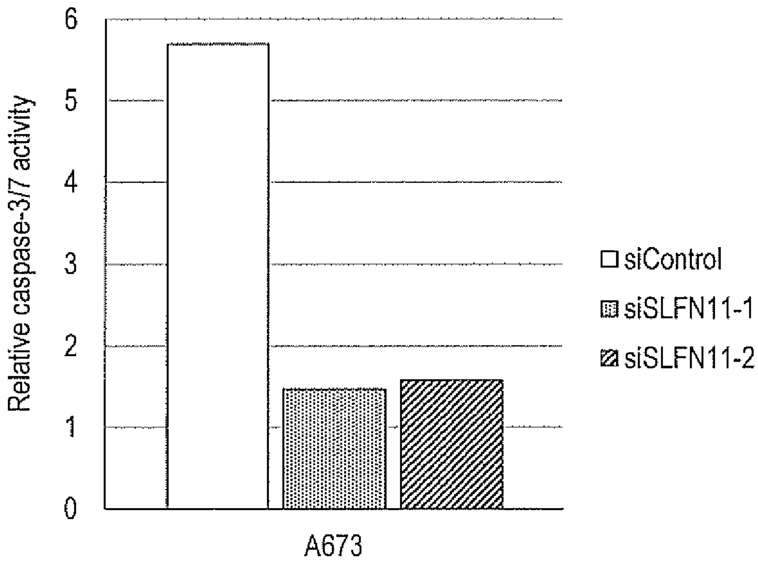
6
5
4
3
2
1
0
Relative caspase-3/7 activity
A673
siControl
siSLFN11-1
siSLFN11-2

[Fig. 16]

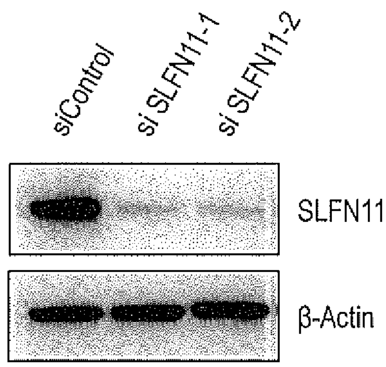
siControl
si SLFN11-1
si SLFN11-2
SLFN11
β-Actin

[Fig. 17]

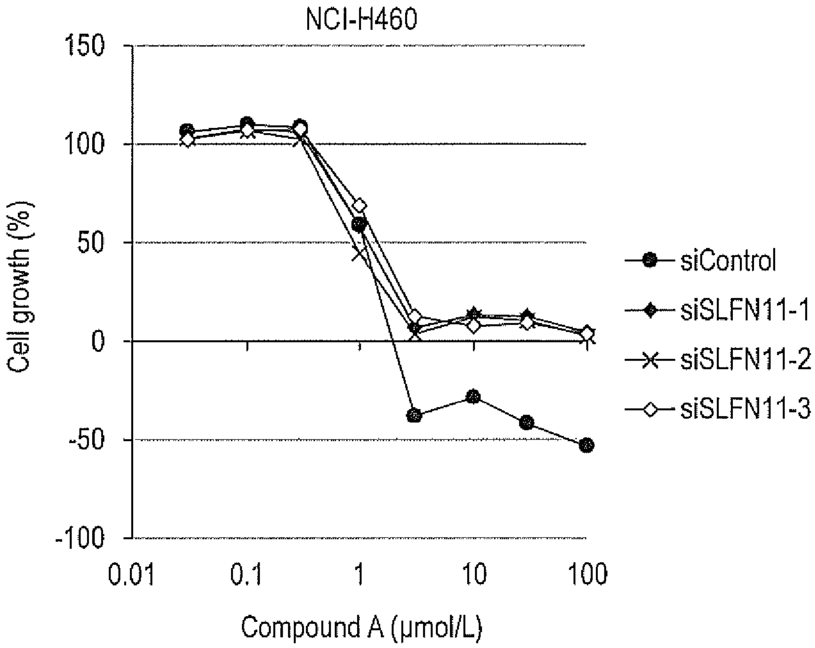
NCI-H460
150
100
50
0
-50
-100
Cell growth (%)
0.01
0.1
1
10
100
Compound A (μmol/L)
siControl
siSLFN11-1
siSLFN11-2
siSLFN11-3

[Fig. 18]

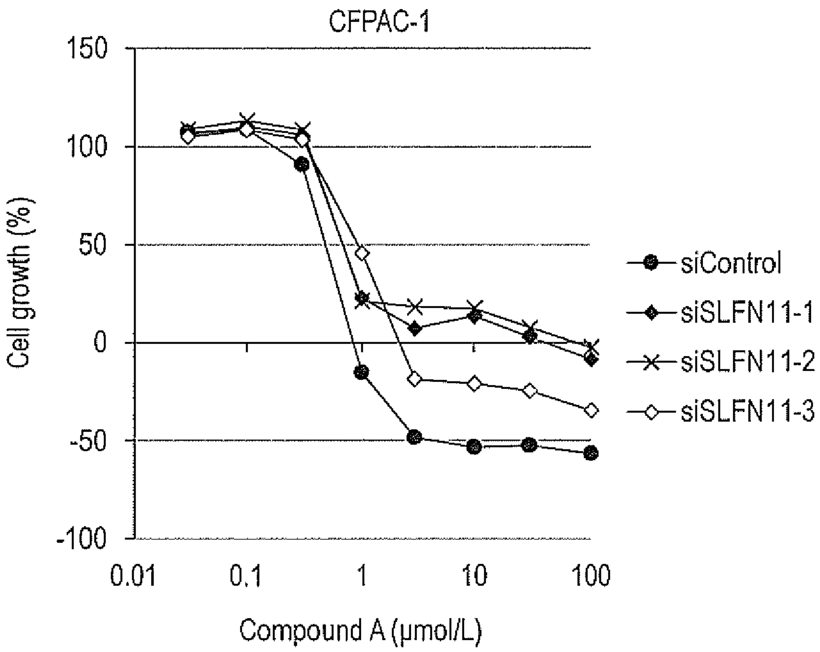
CFPAC-1
150
100
50
0
-50
-100
Cell growth (%)
0.01
0.1
1
10
100
Compound A (μmol/L)
siControl
siSLFN11-1
siSLFN11-2
siSLFN11-3

[Fig. 19]

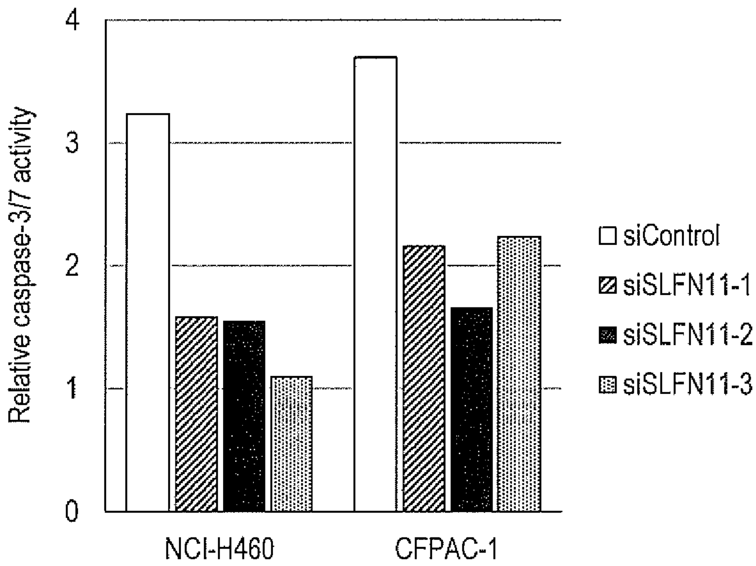
Relative caspase-3/7 activity
0
1
2
3
4
NCI-H460
CFPAC-1
siControl
siSLFN11-1
siSLFN11-2
siSLFN11-3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2020157066 A1
- WO 2017209155 A
- WO 2019106579 A
- WO 2015035223 A
- US 20030162712 A
- US 6413932 B
- US 6727225 B
- US 20020042368 A
- US 5981245 A
- US 5728813 A
- US 5969110 A
- US 6596852 B
- US 6232447 B
- US 6057124 A
- WO 9633172 A
- WO 9627583 A
- EP 1004578 A
- WO 9807697 A
- WO 9803516 A
- WO 9834918 A
- WO 9834915 A
- WO 9833768 A
- WO 9830566 A
- EP 0606046 A
- EP 0931788 A
- WO 9005719 A
- WO 9952910 A
- WO 9952889 A
- WO 9929667 A
- WO 1999007675 A
- EP 1786785 A
- EP 1181017 A
- US 20090012085 A
- US 5863949 A
- US 5861510 A
- EP 0780386 A
- WO 2017068412 A
- WO 06044453 A
- WO 06122806 A
- WO 09036082 A
- WO 09055730 A
- WO 05011700 A
- US 6656963 B
- WO 2012137870 A
- WO 9409010 A
- WO 9802441 A
- WO 0114387 A
- WO 05005434 A
- US 5258389 A
- WO 94090101 A
- WO 9205179 A
- US 5118677 A
- US 5118678 A
- US 5100883 A
- US 5151413 A
- US 5120842 A
- WO 93111130 A
- WO 9402136 A
- WO 9402485 A
- WO 9514023 A
- WO 9516691 A
- WO 9641807 A
- US 5256790 A
- WO 05016252 A


### Non-patent literature cited in the description


- *Annu. Rev. Biochem.*, 1998, vol. 67, 71-98
- *J. Biol. Chem.*, 1970, vol. 245, 5228-5233
- *Nat. Commun.*, 2014, vol. 5, 3128
- *Clin. Sci.*, 2013, vol. 124, 567-578
- *Expert. Opin. Ther. Targets*, 2013, vol. 17, 1423-1437
- *Biochem. Pharmacol.*, 2000, vol. 59, 983-991
- *Biochem. Pharmacol.*, 2009, vol. 78, 1178-11 85
- *Cancer Res.*, 1994, vol. 54, 3686-3691
- *Pharmacol. Rev*, 2005, vol. 57, 547-583
- *Future Oncol.*, 2012, vol. 8, 145-150
- *Oncotarget*, 27 September 2016, vol. 7 (39), 63003-63019
- *Mol. Cell*, 2018, vol. 69 (3), 371-384
- *Am. J. Clin. Pathol.*, 1988, vol. 90 (3), 233-9
- **ALLRED DC et al.** *Mod. Pathol.*, 1998, vol. 11, 155-68
- *Kenbikyo*, 2009, vol. 44 (1), 30-34
- **MODJTAHEDI, H. et al.** *Br. J. Cancer*, 1993, vol. 67, 247-253
- **TERAMOTO, T. et al.** *Cancer*, 1996, vol. 77, 639-645
- **GOLDSTEIN et al.** *Clin. Cancer Res.*, 1995, vol. 1, 1311-1318

- **HUANG, S. M. et al.** *Cancer Res.*, 1999, vol. 59 (8), 1935-40
- **YANG, X. et al.** *Cancer Res.*, 1999, vol. 59, 1236-1243
- **BARNETT et al.** *Biochem. J.*, 2005, vol. 385 (2), 399-408
- **JIN et al.** *Br. J. Cancer*, 2004, vol. 91, 1808-12
- **SARKAR** ; **LI**. *J Nutr.*, 2004, vol. 134 (12), 3493S-3498S
- **DASMAHAPATRA et al.** *Clin. Cancer Res.*, 2004, vol. 10 (15), 5242-52
- **GILLS** ; **DENNIS**. *Expert. Opin. Investig. Drugs*, 2004, vol. 13, 787-97
- **YANG et al.** *Cancer Res.*, 2004, vol. 64, 4394-9
- **HASAKO, S et al.** *Mol Cancer Ther*, 2018, vol. 17, 1648-1658